# FASCICULE DE BREVET EUROPEEN

(11) **EP 3 013 863 B1**
(45) Date de publication et mention de la délivrance du brevet: **13.03.2019**
(21) Numéro de dépôt: 14750247.0
(22) Date de dépôt: 27.06.2014
(51) Int. Cl.: C07K 16/28, G01N 33/53, G01N 33/68, A61P 17/00

(54) **ANTICORPS DIRIGÉS CONTRE LA CANAL SODIQUE NAV1.9 HUMAIN ET LEURS UTILISATIONS POUR LE DIAGNOSTIC DES MALADIES CUTANÉES INFLAMMATOIRES**
ANTIKÖRPER GEGEN MENSCHLICHES NATRIUMKANAL-NAV 1.9 UND VERWENDUNGEN DAVON ZUR DIAGNOSE ENTZÜNDLICHER HAUTERKRANKUNGEN
ANTIBODIES AGAINST HUMAN SODIUM CHANNEL NAV1.9 AND USES THEREOF FOR THE DIAGNOSIS OF INFLAMMATORY SKIN DISEASES

(30) Priorité: 28.06.2013 FR 1356354
(43) Date de publication de la demande: 04.05.2016
(73) Titulaire: Galderma Research & Development, 06410 Biot (FR); Centre National de la Recherche Scientifique, 75016 Paris (FR); Université d'Aix-Marseille, 13007 Marseille (FR)
(72) Inventeur: CREST, Marcel, F-13006 Marseille (FR); DELMAS, Patrick, 13880 Velaux (FR); LONIGRO-RAME, Aurélie, F-13820 Ensues La Redonne (FR); OSORIO, Nancy, F-83240 Cavalaire Sur Mer (FR)
(74) Mandataire: Nederlandsch Octrooibureau
(86) Numéro de dépôt international: PCT/FR2014/051641
(87) Numéro de publication internationale: WO 2014/207400

(56) Documents cités:
- WO-A2-2007/023298
- STRICKLAND IAIN T ET AL: "Changes in the expression of NaV1.7, NaV1.8 and NaV1.9 in a distinct population of dorsal root ganglia innervating the rat knee joint in a model of chronic inflammatory joint pain.", EUROPEAN JOURNAL OF PAIN JUL 2008, vol. 12, no. 5, juillet 2008 (2008-07), pages 564-572, XP002720204, LONDON, ENGLAND ISSN: 1532-2149
- PADILLA ET AL: "Expression and localization of the Nav1.9 sodium channel in enteric neurons and in trigeminal sensory endings: Implication for intestinal reflex function and orofacial pain", MOLECULAR AND CELLULAR NEUROSCIENCES, vol. 35, no. 1, 26 avril 2007 (2007-04-26) , pages 138-152, XP022056623, SAN DIEGO, US ISSN: 1044-7431, DOI: 10.1016/J.MCN.2007.02.008
- LOLIGNIER ET AL: "Nav1.9 channel contributes to mechanical and heat pain hypersensitivity induced by subacute and chronic inflammation", PLOS ONE, vol. 6, no. 8, E23083, 1 août 2011 (2011-08-01), pages 1-11, XP002698323,
- YU YAO-QING ET AL: "Activation of tetrodotoxin-resistant sodium channel NaV1.9 in rat primary sensory neurons contributes to melittin-induced pain behavior.", NEUROMOLECULAR MEDICINE MAR 2013, vol. 15, no. 1, mars 2013 (2013-03), pages 209-217, XP002730891, ISSN: 1559-1174
- FRANK H YU ET AL: "Overview of the voltage-gated sodium channel family", GENOME BIOLOGY (ONLINE), BIOMED CENTRAL LTD, GB, vol. 4, no. 3, 1 janvier 2003 (2003-01-01) , page 207, XP002415109, ISSN: 1465-6914

### SEQUENCE LISTING

<110> Galderma Research & Development
   Centre national de la recherche scientifique
   Université d'Aix-Marseille
<120> Anticorps dirigés contre le canal sodique Nav1.9 humain et leurs utilisations en diagnostic
<130> B1638PC00
<160> 4
<170> PatentIn version 3.5
<210> 1
   <211> 17
   <212> PRT
   <213> Artificial Sequence
<220>
   <223> Fragment de hNav1.9
<400> 1
   Ile Ala Ile Gln Lys Glu Lys Lys Lys Ser Lys Asp Gln Thr Gly Glu
   1               5                   10                  15
   Val
<210> 2
   <211> 16
   <212> PRT
   <213> Artificial Sequence
<220>
   <223> Fragment de hNav1.9
<400> 2
   Glu Ser Gly Lys Asp Gln Pro Pro Gly Ser Asp Ser Asp Glu Asp Cys
   1               5                   10                  15
<210> 3
   <211> 17
   <212> PRT
   <213> Artificial Sequence
<220>
   <223> Fragment de hNav1.9
<400> 3
   Gln Ala Tyr Glu Leu His Gln Glu Asn Lys Lys Pro Thr Ser Gln Arg
   1               5                   10                  15
   Val
<210> 4
   <211> 15
   <212> PRT
   <213> Artificial Sequence
<220>
   <223> Fragment de hNav1.9
<400> 4
   Ser Asn Glu Glu Arg Asn Gly Asn Leu Glu Gly Glu Ala Arg Lys
   1               5                   10                  15

## Description

### Domaine de l'invention

L'invention concerne des anticorps dirigés contre le canal sodique Nav 1.9 et leur utilisation dans le diagnostic des maladies cutanées inflammatoires, en particulier la rosacée.

### Contexte de l'invention

Les maladies cutanées inflammatoires englobent de nombreuses pathologies telles que la rosacée, le psoriasis, l'eczéma de contact, la dermatite atopique, ou encore le prurit.

La rosacée est une dermatose inflammatoire commune chronique et progressive liée à des désordres vasculaires. Elle affecte principalement la partie centrale du visage et se caractérise par un rougissement du visage accompagné de bouffées de chaleur, un érythème facial, de papules, de pustules, de télangiectasie et parfois de lésions oculaires appelées rosacée oculaire. Dans des cas extrêmes, particulièrement chez l'homme, on observe une hypertrophie au niveau nasal appelé rhinophyma. La rosacée survient entre 25 et 70 ans et évolue sur plusieurs années avec des phases de rémission et des phases d'exacerbation. La rosacée est beaucoup plus commune chez les personnes au teint clair et touche particulièrement les femmes. Cependant, les atteintes les plus sévères sont généralement observées chez les hommes. La rosacée est classifiée en 4 sous-types en fonction de diverses caractéristiques cliniques (Wilkin J et al, JAAD, 2002, 46: 584-587) : la rosacée érythématotélangiectasique (sous-type 1), la rosacée papulopustulaire (sous-type 2), la rosacée phymateuse (sous-type 3) et la rosacée oculaire (sous-type 4). Il existe également des formes plus rares de rosacée comme la variante granulomateuse qui est caractérisée par des papules ou nodules indurés jaunes, bruns ou rouges, et par des lésions monomorphes à l'endroit des papules.

Les signes pathologiques de la rosacée varient en fonction du sous-type de la maladie. Néanmoins, on note que les réactions inflammatoires locales et l'hyperactivité vasculaire sont des manifestations constantes de la rosacée.

La pathogénèse de la rosacée est mal connue et peut impliquer plusieurs facteurs. La maladie peut être causée ou favorisée par la présence de microorganismes folliculaires tels que des bactéries et les acariens *Demodex Folliculorum*, une réponse immunitaire innée aberrante, une réactivité vasculaire anormale et une hypersensibilité à des stimuli environnementaux tels que l'exposition aux UVs, les brusques changements de température, la consommation de boissons chaudes, de plats épicés et d'alcool, les fortes émotions (stress, gêne, colère...).

L'état de la technique décrit plusieurs marqueurs utiles pour le diagnostic de la rosacée : il s'agit par exemple de certaines interleukines (WO2013/00872), des récepteurs de chimiokine ou les chimiokines elles-mêmes (WO2013/060865), du récepteur à l'histamine de type 2 (HRH-2) (FR2960152) ou encore d'un récepteur TRPV (Transient Receptor Potential Vanilloid), de préférence TRPV1 (WO2012/084870).

Le psoriasis est une dermatose chronique, évoluant par poussée ou par crise, qui touche environ 2% de la population. Le psoriasis est caractérisé par une hyperprolifération épidermique (renouvellement de l'épiderme accéléré) associée à des troubles de la kératinisation. Les lésions psoriasiques se présentent généralement sous la forme de plaques érythémato-squameuses, souvent prurigineuses. On observe généralement au niveau des lésions une infiltration leucocytaire et une inflammation modérée du derme et de l'épiderme, ce qui laisse penser que le psoriasis serait une maladie autoimmune. Le psoriasis touche fréquemment les zones de frottement comme les genoux, les coudes et la région des lombaires ainsi que le cuir chevelu, les mains et les pieds. On distingue plusieurs formes de psoriasis (psoriasis en gouttes, psoriasis pustuleux...), le psoriasis par plaque (ou psoriasis vulgaire) étant la forme la plus courante. L'étiologie du psoriasis demeure à l'heure actuelle mal connue. Un cas sur deux semble d'origine familiale. Différents gènes de prédisposition ont été découverts (par exemple l'allèle HLA-Cw6). Des études récentes suggèrent que le psoriasis résulte également d'anomalies immunitaires (par exemple implication de l'axe (IL)-23/T-helper (Th)17).

L'eczéma regroupe des dermatoses inflammatoires érythémato-vésiculeuses prurigineuses. Ces pathologies se développent généralement en plusieurs phases : une phase érythémateuses, une phase vésiculeuse, une phase de suintement et une phase de desquamation. On distingue, entre autres, l'eczéma nummulaire, l'eczéma de contact qui est induit par une réaction allergique vis-à-vis d'un agent exogène, et la dermatite atopique.

La dermatite atopique (également appelée dermite atopique ou eczéma atopique) est une dermatose chronique, évoluant par poussées et qui touche essentiellement les enfants, en particulier les nourrissons. La dermatite atopique est caractérisée par une sécheresse cutanée importante (xérose), par des lésions inflammatoires érythémateuses papuleuses ou vésiculeuses, squameuses, avec éventuellement des fissures et une lichénification des lésions L'eczéma atopique touche les convexités des joues, des membres et du cuir chevelu chez les nourrissons. Chez l'enfant plus âgé et l'adulte, les lésions se situent essentiellement au niveau des plis. Tout comme la rosacée et le psoriasis, la pathogénèse de la dermatite atopique est mal connue. Une prédisposition génétique et/ou une anomalie immunitaire est souvent avancée (par exemple un défaut en filaggrin).

Enfin, le prurit est un désordre fonctionnel qui peut être défini comme « une sensation qui provoque le besoin de se gratter ». Il peut être localisé ou généralisé. Il s'agit d'un symptôme fréquent, en particulier des dermatoses inflammatoires avec lésions cutanées telles que le psoriasis et la dermatite atopique. Néanmoins, certains prurits ne sont pas associés à des lésions cutanées spécifiques (prurit « *sine materia* »). Le prurit peut alors être provoqué par une affection générale, être d'origine neurologique ou psychologique ou être associé à une sécheresse cutanée (xérose). Le prurit peut être causé ou aggravé par une hypersensibilité à des facteurs extérieurs (produits chimiques, variations de température et d'humidité, eau calcaire...). Dans certains cas, le prurit est une manifestation neurologique entretenue et/ou amplifiée par une réaction inflammatoire locale résultant du grattement. Il n'existe pas de marqueur général du prurit.

Il existe encore à l'heure actuelle un besoin de nouveaux outils biochimiques et de nouvelles méthodes pour le diagnostic des maladies cutanées inflammatoires, en particulier de la rosacée.

Les canaux sodium voltage-dépendants sont des acteurs essentiels de l'initiation et de la propagation des potentiels d'actions au niveau des cellules dites « excitables ». Ces canaux sodiques sont principalement exprimés au niveau des neurones du système nerveux central et périphérique et au niveau des cellules musculaires. Il s'agit de protéines transmembranaires comprenant une large sous-unité alpha (α) d'environ 260 kDa associée à une ou plusieurs sous-unités béta de 33 à 38 kDa. La sous-unité alpha forme le coeur du canal et comprend quatre domaines membranaires homologues (I-IV), chacun constitué de 6 segments transmembranaires (S1-S6). Les domaines membranaires de la sous unité alpha sont reliés entre eux par de grandes boucles intracellulaires qui comprennent de nombreux sites de régulation. La sous-unité alpha est responsable des propriétés de conductance et de sélectivité du canal alors que les sous-unités béta sont impliquées dans la stabilisation et dans les propriétés cinétiques du canal. A ce jour, 10 isoformes pour la sous-unité alpha ont été identifiées chez l'homme. Le nom attribué à ces isoformes contient le symbole de l'ion transporté (Na), avec en indice l'élément régulateur (le voltage v). Les chiffres qui suivent désignent la sous-famille de gènes et le numéro associé à l'isoforme considérée. A ce jour, la principale sous-famille de gènes identifiée comprend les isoformes Nav1.1 à Nav1.9. Une isoforme supplémentaire, plus éloignée, Nax a été également identifiée. Le pourcentage d'identité de séquence entre les différences isoformes Nav1.1 à Nav 1.9 est d'au moins 60%. Les isoformes Nav1.1 à Nav1.9 se différencient, entre autres, par leur sensibilité à la tétrodotoxine (TTX), un bloqueur très puissant et sélectif des canaux sodiques, et par leur distribution tissulaire. Les isoformes Nav1.1 à Nav1.4, Nav1.6 et Nav1.7 sont dites sensibles à la TTX avec des EC50 de l'ordre du nanomolaire. Les isoformes Nav1.5, Nav1.8 et Nav1.9 sont considérées, quant à elles, comme résistantes à la TTX avec des EC50 de l'ordre du micromolaire. A cet égard, l'EC50 de la TTX pour Nav 1.9 est d'environ 200 µM. En ce qui concerne la distribution tissulaire, les isoformes Nav1.1, Nav1.2, Nav1.3 et Nav1.6 sont principalement exprimées au niveau du système nerveux central. L'isoforme Nav1.4 est exprimée principalement au niveau des myocytes squelettiques alors que Nav 1.5 est essentiellement présente, chez l'individu adulte, au niveau des myocytes cardiaques. Enfin, les isoformes Nav1.7, Nav1.8 et Nav1.9 sont exprimées au niveau du système nerveux périphérique (SNP). Nav1.9 a été principalement détecté au niveau des neurones sensitifs des ganglions spinaux par des expériences d'immunomarquage et par PCR. Nav 1.9 jouerait un rôle dans la perception de la douleur (nociception). Enfin, Nax est exprimé au niveau du coeur, de l'utérus, du muscle lisse, des atrocytes et de certains neurones de l'hypothalamus et du système nerveux central. Pour une revue concernant les canaux sodium voltage-dépendants, on pourra se référer à Yu et Caterall, Genome Biology, 2003, 4 :207 ou Caterall et al., Pharmacological Reviews, 2005, 57:397-409.

Strickland et al, 2008, European Journal of Pain, 12(5) :564-572 divulgue une détection, par l'anticorps K186 polyclonal, de Nav 1.9 au niveau des ganglions spinaux chez un modèle animal de douleur inflammatoire chronique articulaire. A la connaissance des Demandeurs, aucun lien n'a été établi dans l'état de la technique entre l'expression d'une isoforme de Nav, en particulier Nav1.9, et les maladies cutanées inflammatoires.

### Résumé de l'invention

L'invention a pour objet un anticorps dirigé contre Nav 1.9 humain caractérisé en ce que ledit anticorps se lie à un épitope inclus dans un peptide de séquence SEQ ID N°1, SEQ ID N°2 ou SEQ ID N°3. L'anticorps selon l'invention est adapté à une utilisation pour la détection ou la quantification de Nav 1.9 dans un échantillon biologique, de préférence un échantillon de peau. L'anticorps selon l'invention peut être un anticorps polyclonal, de préférence d'isotype IgG ou IgM, ou un anticorps monoclonal. L'anticorps peut être chimérique ou humanisé. Il peut être choisi parmi une immunoglobuline comprenant 2 chaînes lourdes et 2 chaînes légères, un anticorps à simple domaine (sdab), ou une IgNar, un polypeptide comprenant un domaine variable d'un anticorps tel qu'un ScFv, un VH, un V_{H}H, un V_{NAR}, un Fab, et les versions chimériques ou humanisés de ceux-ci.

Dans certains modes de réalisation, l'anticorps selon l'invention ne se lie pas à Nav 1.5 et Nav 1.7, de préférence humain. Dans un mode de réalisation supplémentaire, l'anticorps selon l'invention est obtenu à partir d'anticorps, ou de cellules exprimant des anticorps, provenant d'un animal non-humain immunisé avec un composé antigénique comprenant au moins un peptide de SEQ ID N°1, SEQ ID N°2 ou SEQ ID N°3.

L'anticorps selon l'invention peut être couplé à un moyen de détection, de préférence choisi parmi une enzyme produisant un signal détectable telle que la peroxydase de raifort, la phosphatase alcaline, la 3-galactosidase, ou la glucose-6-phosphate déshydrogénase, un chromophore, un composé fluorescent, un composé luminescent, un radionucléide tel que ³²P, ³⁵S ou ¹²⁵I, une particule métallique, par exemple une nanoparticule d'or, une biotine, une streptavidine, une avidine, un sucre ou une lectine.

Un objet supplémentaire de l'invention est une méthode de préparation d'un anticorps dirigé contre Nav 1.9 humain comprenant une étape d'immunisation d'un animal non-humain avec un composé antigénique comprenant au moins un peptide de SEQ ID N°1, SEQ ID N°2 ou SEQ ID N°3. Le composé antigénique peut en outre comprendre une protéine porteuse couplée à une ou plusieurs copies dudit peptide.

Dans certains modes de réalisation, la méthode de préparation selon l'invention peut comprendre, en outre, la récupération de l'anticorps dirigé contre Nav 1.9 humain à partir du plasma sanguin de l'animal non-humain, après immunisation.

Dans d'autres modes de réalisation, la méthode de préparation selon l'invention peut comprendre, la production de l'anticorps dirigé contre Nav 1.9 par un hybridome obtenu à partir d'une cellule exprimant des anticorps, de préférence un lymphocyte B, provenant de l'animal non-humain, après immunisation.

Un objet supplémentaire selon l'invention est un anticorps susceptible d'être obtenu par ladite méthode de préparation.

L'invention a également pour objet l'utilisation d'un anticorps selon l'invention pour le diagnostic, le pronostic et/ou la prédiction *in vitro* d'une maladie cutanée inflammatoire.

Un objet supplémentaire est l'utilisation d'un anticorps selon l'invention pour le suivi *in vitro* de l'évolution d'une maladie cutanée inflammatoire ou pour évaluer *in vitro* l'efficacité d'un médicament pour le traitement d'une maladie cutanée inflammatoire.

Dans certains modes de réalisation, l'anticorps selon l'invention est utilisé pour détecter ou quantifier Nav 1.9 dans un échantillon de peau d'un patient. Dans un mode de réalisation supplémentaire, la maladie cutanée inflammatoire est choisie parmi le groupe constitué par la rosacée, le psoriasis, le prurit, l'eczéma de contact et la dermatite atopique. De préférence, il s'agit de la rosacée, en particulier choisie parmi le groupe constitué par la rosacée érythèmatotélangiectasique (sous-type I), la rosacée papulopustuleuse (sous-type II), la rosacée phymateuse (sous-type III), la rosacée oculaire (sous-type IV) et une variante granulomateuse de la rosacée. Dans un mode de réalisation avantageux, Nav 1.9 est détecté ou quantifié par immunodosage, par western blot ou par immunomarquage.

Enfin, l'invention a également pour objet un kit pour le diagnostic d'une maladie cutanée inflammatoire, de préférence la rosacée, comprenant au moins un anticorps selon l'invention et en option un moyen de détection dudit anticorps.

### Figures

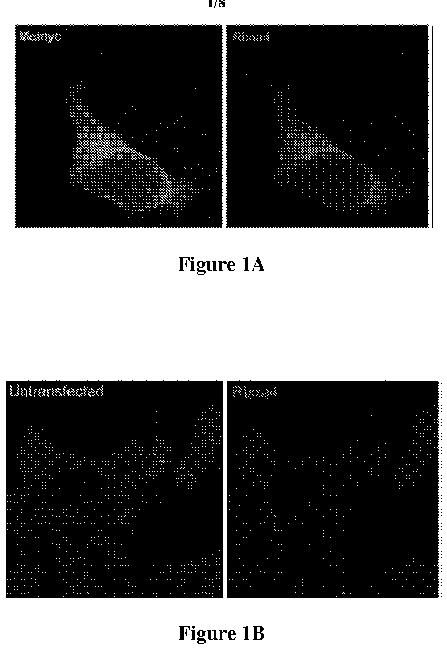

La **Figure 1** montre les résultats d'immunomarquage de cellules HEK après fixation au PAF (paraformaldéhyde) 2% par un anticorps monoclonal anti-Myc et l'anticorps anti-hNav 1.9 Rbαa4. **Figure 1a** : cellules transfectées avec phNav1.9-myc. **Figure1b** : cellules non transfectées.

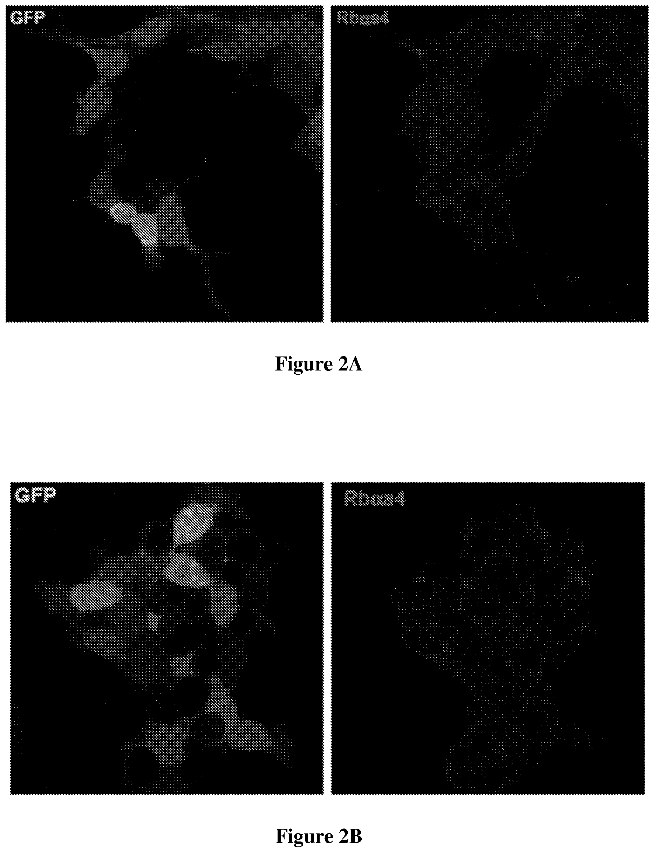

La **Figure** 2 montre les résultats de marquage avec l'anticorps Rbαa4 et de visualisation par la GFP pour les cellules co-transfectés par hNav 1.5 et la GFP (Figure 2A) ou par hNav 1.7 avec la GFP (Figure 2B). Alors que le signal émis par la GFP est clairement visible, le marquage avec Rbαa4 des cellules est très faible voire inexistant.

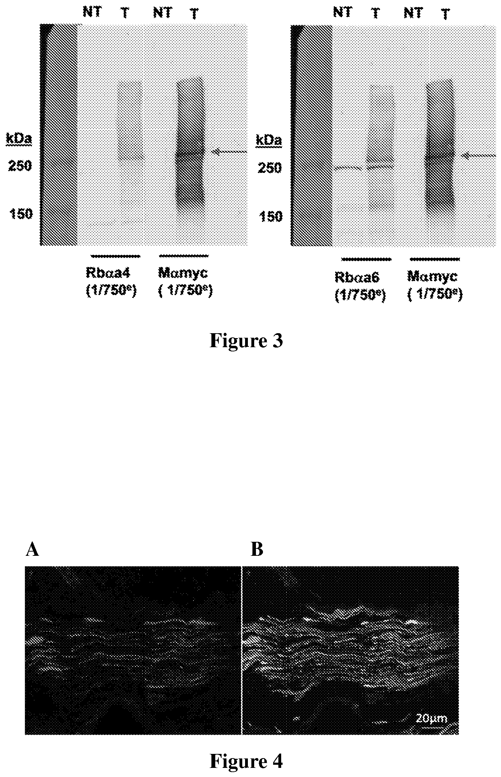

La **Figure 3** montre les western blot réalisés sur des extraits protéiques de cellules HEK non transfectées ou transfectées avec un plasmide codant pour hNav 1.9-myc. NT : lysat de cellules non-transfectées (témoin négatif), T : lysat de cellules transfectées ; L'anticorps Rbαa6 reconnaît une bande protéique de poids moléculaire 250 kDa qui n'est pas détectée pour les cellules non transfectées . Un co-marquage par l'anticorps anti-Myc est observé.
La **Figures 4** montre les résultats d'immunomarquage d'une coupe de derme profond avec l'anticorps anti-Nav1.9 Rbαa6 (Figure 4A)). Le co-marquage avec un anticorps anti-périphérine est illustré à la figure 4B. On observe une expression localisée de Nav 1.9 au niveau des fibres sensitives du derme.

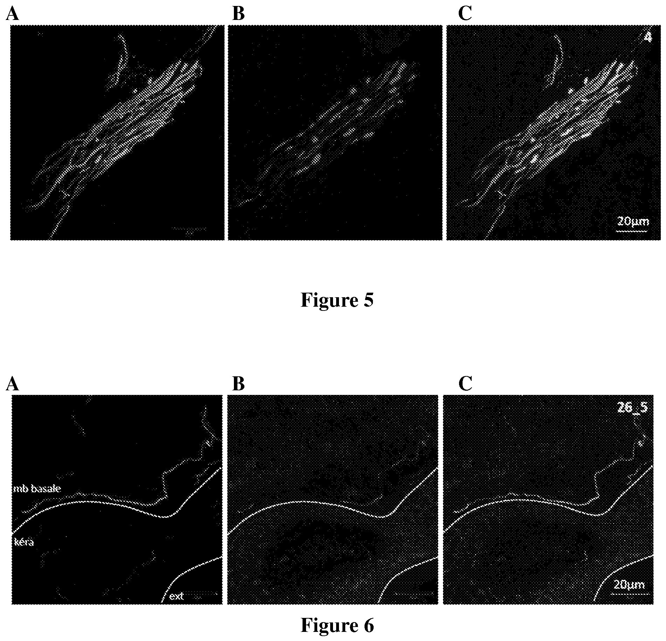

La **Figure 5** montre les résultats d'immunomarquage d'une coupe de derme profond avec l'anticorps anti-Navl.9 de référence 3881.1 (Figure 5B) et un anticorps anti-périphérine (Figure 5A). Le co-marquage est montré à la figure 5C. On observe également une détection de Nav 1.9 au niveau des fibres sensitives du derme.
La **Figure 6** montre l'expression localisée de Nav1.9 au niveau d'une fibre nerveuse isolée du derme, à proximité de l'interface épiderme-derme. La figure 6A montre le marquage avec l'anticorps anti-périphérine, la figure 6B montre le marquage avec l'anticorps 3881. Le comarquage de la périphérine et de Nav 1.9 est illustré à la figure 6C. Mb basale : membrane basale, kéra : kératinocyte, Ext : extérieur.

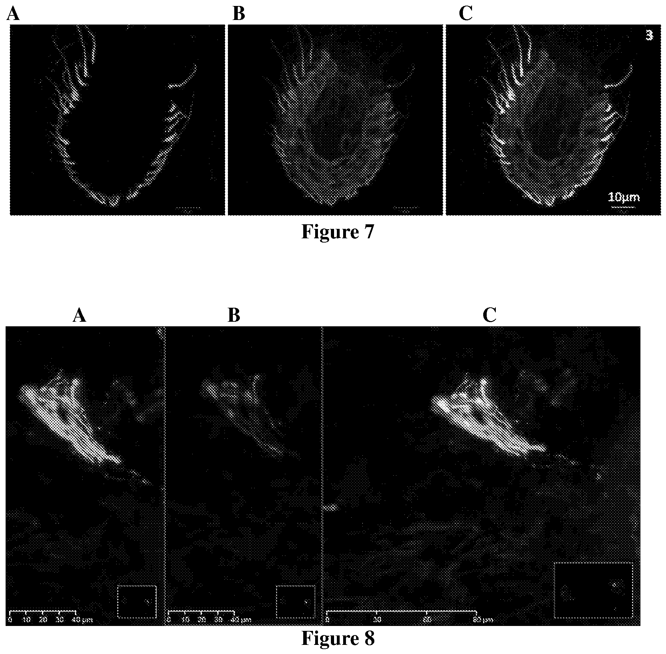

La **Figure 7** montre l'expression localisée de Nav 1.9 au niveau des fibres sensitives innervant le follicule pileux. La figure 7A montre le marquage avec l'anticorps anti-périphérine, la figure 7B montre le marquage avec l'anticorps 3881. Le co-marquage de la périphérine et de Nav 1.9 est illustré à la figure 7C.

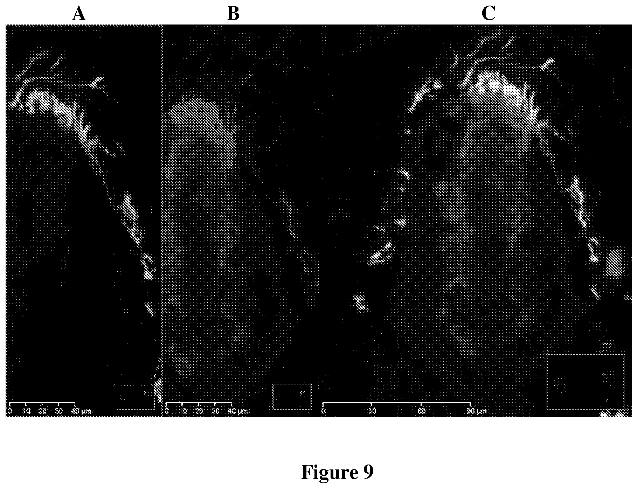

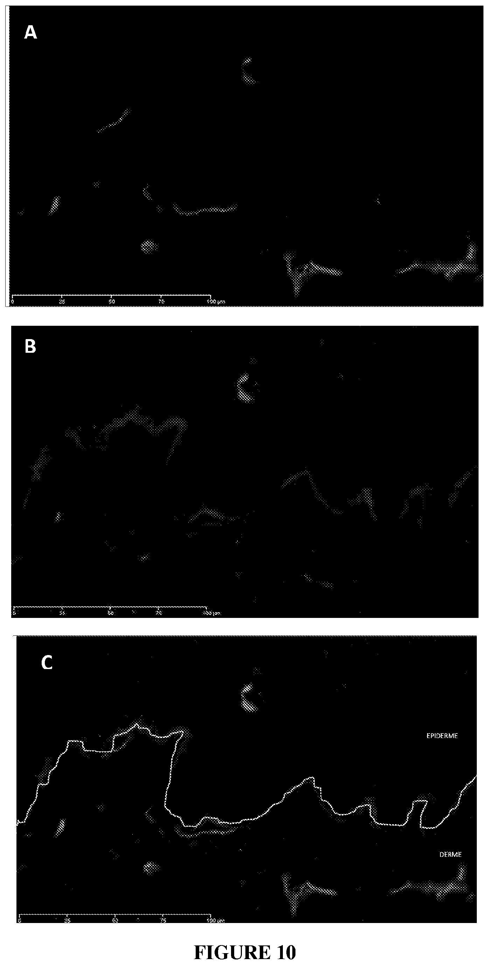

Les **Figures 8****,** **9** et **10** montrent l'immunomarquage d'échantillons cutanés de patients souffrant de rosacée. La Figure 8 correspond à une coupe de derme et la Figure 9 montre un follicule pileux. La Figure 10 présente quant à elle une coupe de derme-épiderme. Figures 8A, 9A et 10A : marquage avec l'anticorps anti-périphérine. Figures 8B, 9B et 10B : montre le marquage avec l'anticorps 3881.1. Le comarquage est montré dans les Figures 8C, 9C et 10C.

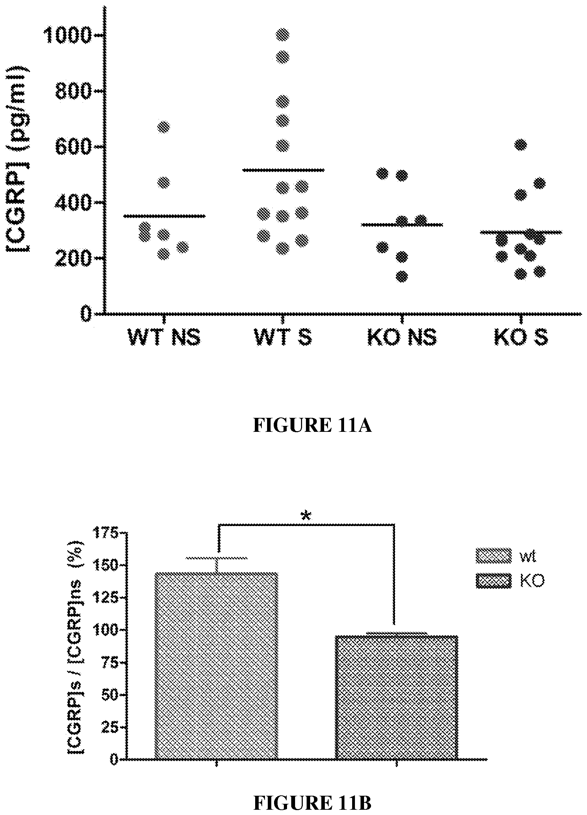

Les **Figure 11A et 11B** montrent les résultats des expériences de libération de CGRP (calcitonin gene-related peptide) chez des cultures primaires de neurones, issus de ganglions spinaux de souris sauvages (WT) ou invalidées pour le gène de Nav1.9 (KO), après traitement (S) à la capsaïcine (15 min, 300 nM) ou sans traitement à la capsaïcine (NS) (voir Example 3 ci-après).
La **Figure 11A** montre les concentrations de CGRP (pg/ml) détectées dans le surnageant de chaque culture cellulaire testée ainsi que les concentrations moyennes de CGRP pour chaque groupe d'expériences (barre noire). La **Figure 11B** montre les rapports (en %) de la concentration moyenne de CGRP dans le surnageant, après stimulation, sur la concentration moyenne, avant stimulation, pour les cultures cellulaires provenant de souris WT et KO, respectivement. On observe une augmentation d'environ 43,5% de la concentration de CGRP après traitement à la capsaïcine, pour les cultures cellulaires obtenues à partir des souris WT. En revanche, aucune augmentation n'est observée pour les cultures neuronales provenant de souris KO, après stimulation à la capsaïcine.

### Description détaillée de l'invention

Les Demandeurs ont préparé trois anticorps polyclonaux - dénommés ci-après Rbαa4, 3881-1 et Rbαa6 - dirigés contre Nav 1.9 humain. Ces anticorps ont été obtenus par immunisation d'un animal non-humain (lapin) avec un composé antigénique comprenant un peptide de séquence SEQ ID N°1, SEQ ID N°2 ou SEQ ID N°3. Le peptide de séquence SEQ ID N°1 correspond à un fragment de la partie N-terminale de Nav 1.9 humain alors que les peptides de séquence SEQ N°2 et SEQ ID N°3 sont des fragments provenant des boucles intracellulaire reliant les domaines transmembranaires I-II et II-III de hNav 1.9, respectivement. Les Demandeurs ont mis en évidence que ces anticorps polyclonaux permettaient de détecter *in vitro* la présence de Nav 1.9 et ceci de manière spécifique. Il s'est avéré que ces anticorps permettaient également de détecter l'expression de Nav 1.9 au niveau de tissus humains connus pour exprimer ce canal sodium, par exemple au niveau de la pulpe dentaire et des neurones myentériques (côlon). Les anticorps polyclonaux préparés par les Demandeurs se sont révélés être des outils biochimiques fiables et sensibles pour explorer la distribution tissulaire de Nav 1.9

A l'issue de longues recherches, les Demandeurs ont démontré, par des expériences d'immunomarquage réalisées grâce aux anticorps Rbaa4, 3881-1 et Rbaa6, que Nav1.9 était exprimé, localement, au niveau des fibres sensitives de la peau (Exemple 3). Par ailleurs, il s'est avéré que le canal sodique Nav 1.9 était plus fortement exprimé dans les échantillons de peau provenant de patients atteints par la rosacée que dans les échantillons de peau provenant d'individus sains (Exemple 4). En parallèle, les Demandeurs ont montré que le canal sodique Nav 1.9 était impliqué dans les mécanismes de relargage de peptides pro-inflammatoires par les neurones sensitifs, consécutivement à leur stimulation (Exemple 5). Ces résultats suggèrent très fortement une implication de Nav1.9 dans la potentialisation de la réponse inflammatoire résultant de la stimulation des fibres sensitives de la peau. Les Demandeurs ont donc montré, pour la première fois, que Nav1.9 est un marqueur de choix pour le diagnostic des maladies cutanées inflammatoires, en particulier la rosacée.

Ainsi, la présente invention concerne des anticorps dirigés contre Nav 1.9 humain, leur préparation et leur utilisation dans le diagnostic.

### ▪ Anticorps dirigés contre Nav 1.9

Selon un premier aspect, l'invention concerne un anticorps dirigé contre Nav 1.9 humain (également désigné ici hNav 1.9 et Nav 1.9). On entend par « Nav1.9 » la sous-unité alpha humaine des canaux sodiques voltage-dépendant codée par le gène SCN11A, également dénommé NaN, SCN12A et SNS-2 dans la littérature. Le numéro d'identifiant du gène Nav 1.9 humain dans la base de gènes du NCBI est 11289 (NCBI Gene ID). La séquence protéique de Nav 1.9 humain est notamment décrite dans la base de séquences UniProt (Référence de séquence : Q9UI33) et celle du NCBI (référence de séquence NCBI : NP_054858). En ce qui concerne l'ARNm pour Nav 1.9, voire la séquence de référence NCBI : NM_014139. L'anticorps selon l'invention est caractérisé en ce qu'il est capable de se lier à un épitope inclus dans un peptide de SEQ ID N°1, de SEQ ID N°2 ou de SEQ ID N°3.

Le peptide de séquence SEQ ID N°1 correspond à un fragment de la partie N-terminale de hNav 1.9 (acides aminés de la position 31 à 47 de hNav1.9) alors que les peptides de séquence SEQ N°2 et SEQ ID N°3 sont des fragments provenant des boucles intracellulaire reliant les domaines membranaires I-II et II-III de hNav 1.9, respectivement. Plus précisément, le peptide de SEQ ID N°2 correspond au fragment allant de l'acide aminé 473 à l'acide aminé 488 de la séquence de hNav1.9 et le peptide de SEQ ID N°3 correspond au fragment allant de l'acide aminé 945 à l'acide aminé 961 de la séquence de Nav 1.9.

On entend par « anticorps anti-Nav 1.9 » ou par « anticorps dirigé contre Nav 1.9 » tout anticorps ou tout polypeptide comprenant un fragment d'anticorps capable de reconnaitre et de se lier spécifiquement à un épitope de la sous-unité alpha Nav 1.9 humain, de préférence ayant une séquence en acides aminés incluse dans SEQ ID N°1, SEQ ID N°2 ou SEQ ID N°3.

Nav 1.9 peut être associé à d'autres protéines, par exemple à une sous-unité beta, ou exprimé au niveau d'une membrane cellulaire ou encore sous une forme isolée (par exemple sous une forme pré-purifiée, purifiée et/ou non-enchâssée dans une membrane cellulaire).

Dans un mode de réalisation préféré, l'anticorps selon l'invention est capable de se lier à Nav 1.9 exprimé au niveau d'une membrane cellulaire.

Les Demandeurs ont montré que les anticorps selon l'invention sont particulièrement adaptés à une utilisation pour la détection ou la quantification de Nav 1.9 dans un échantillon biologique, de préférence un échantillon tissulaire, par exemple un échantillon de peau. Les anticorps selon l'invention peuvent être utilisés pour détecter ou quantifier Nav 1.9 par des techniques d'immunomarquage, et/ou d'immunodosage et/ou par western blot.

Sans vouloir être lié par une quelconque théorie, les Demandeurs sont d'avis que les propriétés de détection des anticorps selon l'invention résultent des épitopes spécifiques de Nav 1.9 contre lesquels ils sont dirigés. En effet, les Demandeurs ont montré que des anticorps dirigés contre d'autres régions de Nav 1.9 - par exemple contre le peptide de SEQ ID N°4 de la boucle intracellulaire liant les domaines membranaires II-III de Nav 1.9 - présentent soit une sensibilité trop faible (c'est-à-dire une affinité trop faible pour Nav 1.9) et/ou une spécificité trop faible pour être utilisés pour la détection ou la quantification tissulaire de Nav 1.9.

Dans certains modes de réalisations, l'anticorps selon l'invention a été obtenu à partir d'un anticorps de préférence plasmatique, ou d'une cellule exprimant des anticorps, ledit anticorps ou cellule provenant d'un animal non-humain immunisé avec un composé antigénique comprenant au moins un peptide de SEQ ID N°1, SEQ ID N°2 ou SEQ ID N°3.

De préférence, le composé antigénique comprend un peptide de séquence SEQ ID N°1, SEQ ID N°2 ou SEQ ID N°3. On entend par « composé antigénique » un composé dont l'administration à un animal non-humain, seule ou en combinaison avec un immunoadjuvant, est capable d'induire une réaction immunitaire humorale. Dans un mode de réalisation préféré, le composé antigénique comprend plusieurs copies dudit peptide, éventuellement couplées entre elles. Dans certains modes de réalisation, le composé antigénique est une protéine porteuse sur laquelle est greffée une ou plusieurs copies d'un peptide ou de plusieurs peptides tels que définis précédemment. Des méthodes pour obtenir un anticorps selon l'invention sont décrites en détails ci-après.

Comme mentionné ci-avant, un anticorps selon l'invention peut être tout anticorps ou polypeptide comprenant un fragment d'anticorps capable de reconnaitre et de se lier spécifiquement à Nav 1.9 humain, de préférence à un épitope inclus dans une région de Nav 1.9 de séquence choisie parmi SEQ ID N°1, SEQ ID N°2 et SEQ ID N°3. L'anticorps selon l'invention peut être un anticorps monoclonal ou polyclonal. On entend, par « anticorps polyclonaux » ou « anticorps polyclonal », un mélange d'anticorps dirigés contre plusieurs épitopes. On entend par « anticorps monoclonal » ou « anticorps monoclonaux », un anticorps ou un ensemble d'anticorps qui reconnaisse un même et unique épitope.

L'anticorps selon l'invention peut être un anticorps humanisé ou chimérique. Dans certains modes de réalisations, il est préparé par un procédé incluant une étape d'immunisation d'un animal non-humain. A titre alternatif, il peut être préparé par des techniques de génie génétique. Dans certains modes de réalisation, l'anticorps est une immunoglobuline « entière » c'est-à-dire qu'il a la structure d'une immunoglobuline présente naturellement dans un plasma sanguin. Il peut s'agir d'une immunoglobuline comportant 2 chaînes lourdes et 2 chaînes légères, par exemple une immunoglobuline d'isotype G ou d'un anticorps à chaîne lourde, c'est-à-dire constitué de 2 chaînes lourdes dépourvu de chaînes légères tel qu'un anticorps à chaîne lourde d'un camélidé (HcAb) ou une IgNAR d'un poisson cartilagineux.

Dans d'autres modes de réalisation, l'anticorps selon l'invention est un polypeptide comprenant un fragment d'une immunoglobuline, de préférence un domaine variable capable de se lier à Nav 1.9, en particulier à un épitope inclus dans un peptide de séquence SEQ ID N°1, SEQ ID N°2 et SEQ ID N°3. L'anticorps selon l'invention peut être choisi parmi un ScFv, un VH, un V_{H}H (également appelé anticorps à simple domaine), un V_{NAR}, un Fab, ou un Fab2, les versions chimériques et humanisés de ceux-ci et les polypeptides les comprenant. A titre d'exemple, l'anticorps selon l'invention peut être une protéine de fusion comprenant un domaine ScFv humanisé fusionné avec les domaines CH3-CH2 du domaine Fc d'une IgG humaine.

Dans un mode de réalisation préféré, l'anticorps selon l'invention est un anticorps polyclonal. Dans un mode de réalisation supplémentaire, l'anticorps est spécifique de Nav 1.9, en particulier par rapport à Nav 1.5 et/ou Nav 1.7. Ceci signifie que l'affinité de l'anticorps selon l'invention pour Nav 1.9 est supérieure celle observée pour une autre protéine, en particulier Nav 1.5 et/ou Nav 1.7.

La spécificité de l'anticorps selon l'invention pour Nav 1.9 peut être déterminée par toute méthode connue de l'homme du métier. Lorsque l'anticorps selon l'invention est monoclonal, on peut comparer par exemple sa constante de dissociation (Kd) pour Nav 1.9 par rapport à son éventuel Kd pour l'autre protéine. Les constantes de dissociation peuvent être déterminées par toute méthode adaptée, y compris par résonance plasmonique de surface. On peut considérer que l'anticorps selon l'invention est spécifique de Nav 1.9 par rapport à une autre protéine si sa constante de dissociation pour Nav 1.9 est au moins 10 fois inférieure, de préférence au moins 100 voire au moins 1000 fois inférieure, à celle observée pour l'autre protéine.

De manière générale, le Kd d'un anticorps monoclonal selon l'invention est généralement inférieur à 10⁻⁶, de préférence 10⁻⁹ M.

Lorsque l'anticorps selon l'invention est polyclonal, on peut évaluer sa spécificité vis-à-vis de Nav 1.9 et d'une autre protéine par immunomarquage, comme cela est illustré dans les exemples. A cette fin, on effectue un immunomarquage avec l'anticorps selon l'invention d'une première culture cellulaire exprimant de manière recombinante le Nav 1.9 humain, et d'une seconde culture cellulaire exprimant de manière recombinante l'autre protéine.

On considère que l'anticorps est spécifique de Nav 1.9 par rapport à l'autre protéine si le marquage obtenu pour la première culture cellulaire exprimant Nav 1.9 est plus important (d'au moins 20%, de préférence d'au moins 50% en intensité et/ou en surface) par rapport à celui obtenu pour la seconde culture cellulaire. A titre alternatif, la spécificité d'un anticorps peut être évaluée grâce à des expériences de western blot effectuées sur des extraits protéiques provenant des systèmes cellulaires ci-dessus mentionnés.

Dans certains modes de réalisation, l'anticorps selon l'invention ne lie pas Nav 1.5 et/ou Nav.1.7. En d'autres termes, le rapport de sa constante de dissociation pour Nav 1.9 sur celle de Nav1.5 et/ou Nav1.7 est au plus égale à 10⁻³, de préférence 10⁻⁴ et/ou le marquage avec l'anticorps selon l'invention d'une culture cellulaire exprimant Nav 1.5 et/ou Nav 1.7 (sans exprimer Nav 1.9) est faible, voire inexistant, en comparaison avec le marquage obtenu pour une lignée cellulaire identique exprimant Nav 1.9 (sans exprimer Nav 1.5 ou Nav 1.7).

Dans d'autres modes de réalisation l'anticorps selon l'invention ne présente pas une réactivité croisée vis-à-vis d'une protéine homologue à Nav 1.9 humain. Par exemple, l'anticorps selon l'invention ne lie pas Nav 1.9 de rat, de lapin ou de souris.

Dans un mode de réalisation supplémentaire, l'anticorps selon l'invention ne présente pas de réactivité croisée vis-à-vis de hNav 1.8, et/ou hNav 1.7 et/ou hNav 1.5, et/ou des Nav 1.9 non-humains.

Dans un autre mode de réalisation, l'anticorps dirigé contre Nav 1.9 est couplé à un moyen permettant sa détection. Le moyen de détection peut être tout moyen connu par l'homme du métier. Il peut s'agir d'une enzyme produisant un signal détectable par exemple par colorimétrie, fluorescence, luminescence, comme la peroxydase de raifort, la phosphatase alcaline, la 3-galactosidase, ou la glucose-6-phosphate déshydrogénase. A titre alternatif, l'anticorps peut être couplé à un chromophore, par exemple, un composé fluorescent, luminescent ou colorant. A titre d'exemple de molécules fluorescentes, on peut citer les Alexa ou les phycocyanines. Le moyen de détection peut être également choisi parmi les radionucléides tels que ³²P, ³⁵S ou le ¹²⁵I, les particules métalliques, par exemple, les nanoparticules d'or ou encore les quantum dots. La détection de l'anticorps peut être également effectuée grâce à un système de reconnaissance de type biotine/streptavidine ou avidine ou encore sucre/lectine. Dans ce cas, l'anticorps peut être, par exemple, couplé à la biotine et la détection se fait grâce à la streptavidine ou l'avidine elle-même marquée par un moyen de détection tels que ceux précédemment cités.

Dans d'autres modes de réalisation, l'anticorps selon l'invention n'est pas couplé à un moyen de détection. Dans ce cas, l'anticorps selon l'invention peut être détecté de manière indirecte en utilisant un anticorps de détection (également appelé anticorps secondaire) lui-même couplé à un moyen de détection comme décrit précédemment. L'anticorps de détection peut être par exemple dirigé contre la partie Fc de l'anticorps selon l'invention.

### ▪ Méthode de préparation d'un anticorps selon l'invention

Un objet supplémentaire selon l'invention est une méthode de préparation d'un anticorps dirigé contre Nav 1.9 humain. Cette méthode peut comprendre une étape d'immunisation d'un animal non-humain avec un composé antigénique comprenant t un peptide de SEQ ID N°1, N°2 ou N°3.

L'animal non-humain peut être tout animal utilisé pour générer des anticorps en laboratoire ou dans l'industrie pharmaceutique. Il peut s'agir, par exemple, d'une souris, d'un rat, une chèvre ou d'un lapin. Pour obtenir un anticorps à chaîne lourde, l'animal non-humain est choisi parmi un camélidé ou un poisson cartilagineux.

De préférence, le composé antigénique comprend au moins un peptide, tel que défini ci-avant, de préférencede SEQ ID N°1, SEQ N°2 ou SEQ ID N°3. Dans certains modes de réalisation, le composé antigénique peut comprendre plusieurs peptides, par exemple un peptide de séquence SEQ ID N°1 et un peptide de séquence SEQ ID N°2, ou un peptide de SEQ ID N°2 et un peptide de SEQ ID N°3, ou encore un peptide de SEQ ID N°1 et un peptide de SEQ ID N°3.

Dans certains modes de réalisation, le composé antigénique comprend une protéine porteuse couplée à une ou plusieurs copies dudit peptide ou desdits peptides tels que définis ci-avant.

La protéine porteuse peut être choisie par exemple parmi la KLH (Keyhole limpet hemocyanin), une albumine sérique, une ovalbumine, une poly-L-lysine ou un toxoïde, par exemple le toxoïde tétanique. L'étape d'immunisation de l'animal non-humain comprend l'administration à l'animal non-humain du composé antigénique, seul ou en combinaison avec un immunoadjuvant. L'immuno-adjuvant peut être tout immunoadjuvant classiquement utilisé en recherche, par exemple, l'adjuvant de Freund, une huile minérale comme le specol, un squalène ou un sel d'aluminium. L'administration du composé antigénique peut être faite par toute voie, de préférence par voie sous-cutanée, intramusculaire ou intra-péritonéale. La dose du composé antigénique varie selon l'animal-non humain à immuniser et est comprise généralement entre 10 µg et 10 mg. L'administration du composé antigénique peut être renouvelée plusieurs fois, par exemple 2, 3, 4, 5, 6 fois jusqu'à obtenir une réponse immunitaire humorale satisfaisante. Chaque administration peut être espacée d'un ou plusieurs jours, typiquement de 7 à 30 jours. Les étapes de la méthode de préparation selon l'invention varie selon que l'on souhaite obtenir un anticorps monoclonal, polyclonal ou encore un anticorps chimérique ou humanisé.

Les anticorps monoclonaux peuvent être obtenus à partir de cellules productrices d'anticorps, par exemple des lymphocytes B, prélevées après immunisation de l'animal non-humain. Dans ce cas, on utilise la technique classique de préparation d'hybridomes bien connue de l'homme du métier.

Un anticorps polyclonal peut être obtenu à partir du plasma sanguin de l'animal non-humain, prélevé après immunisation, en mettant en oeuvre des étapes classiques de purification comme des étapes de centrifugation, de précipitation et de chromatographies d'affinité, en particulier en utilisant comme ligand d'affinité un peptide dérivé de Nav 1.9 présent dans le composé antigénique utilisé pour immuniser l'animal non-humain ou la protéine hNav 1.9 elle-même. On préfère utiliser une chromatographie d'affinité dans laquelle le ligand d'affinité est choisi parmi le groupe constitué par le peptide de séquence SEQ ID N°1, le peptide de séquence SEQ ID n°2 et le peptide de séquence SEQ ID N°3.

Selon un aspect supplémentaire, il est égalemnt décrit ici une méthode de sélection d'un anticorps dirigé contre hNav 1.9 adapté à une utilisation pour la détection ou la quantification de hNav 1.9 dans un échantillon biologique, de préférence un échantillon tissulaire, par exemple un échantillon de peau, ladite méthode de sélection comprenant les étapes consistant à :
(a) fournir un ou plusieurs anticorps susceptibles d'être dirigé contre hNav 1.9,
(b) mettre en contact le ou les anticorps fournis à l'étape (a) avec un peptide ayant au moins 90%, de préférence 100%, d'identité de séquence avec une séquence choisie parmi SEQ ID N°1, SEQ ID N°2 et SEQ ID N°3, ledit peptide étant de préférence immobilisé sur un support, et
(c) sélectionner le ou les anticorps ayant formé un complexe avec le peptide à l'étape (b).

Le ou les anticorps de l'étape (a) peuvent être fournis sous la forme d'une solution comprenant un mélange d'anticorps, par exemple une solution dérivant d'un plasma sanguin, sous une forme isolée (purifiée ou pré-purifiée), chaque anticorps étant testé séparément, ou encore sous la forme d'une librairie d'anticorps exprimée à la surface de phages ou de cellules.

Il est également décrit ici un anticorps dirigé contre Nav 1.9 obtenu ou susceptible d'être obtenu par l'une des méthodes de préparation ci-dessus décrites.

### ▪ Utilisations de l'anticorps selon l'invention

Comme mentionné précédemment, les anticorps selon l'invention sont particulièrement adaptés pour une utilisation en tant qu'outil diagnostique pour la détection et/ou la quantification de Nav 1.9 dans des échantillons biologiques. A cet égard, les Demandeurs ont montré que les maladies cutanées inflammatoires, en particulier la rosacée, se caractérisent par une surexpression de Nav 1.9 au niveau des fibres sensitives de la peau.

Ainsi, un objet supplémentaire selon l'invention est l'utilisation d'un anticorps selon l'invention pour le diagnostic, le pronostic et/ou la prédiction d'une maladie cutanée inflammatoire.

Au sens de l'invention, une « pathologie cutanée inflammatoire » ou une « maladie cutanée inflammatoire » désigne une maladie ou un désordre affectant la peau caractérisée en ce qu'elle est associée, causée ou peut impliquer une réaction inflammatoire. La pathologie inflammatoire cutanée peut affecter n'importe quelle partie de la peau, y compris le visage, le cuir chevelu et la région oculaire, en particulier les paupières. Dans certains modes de réalisation, la réaction inflammatoire est une inflammation neurogène locale c'est-à-dire déclenchée, au moins partiellement, par une réponse neurologique, en particulier par la libération locale de neuropeptides pro-inflammatoires tels que la substance P et le peptide CGRP (calcitonin gene-related peptide).

Dans d'autres modes de réalisation, la maladie cutanée inflammatoire est initiée par un désordre neurologique, comme cela peut être observé dans certains cas de prurit.

Les pathologies inflammatoires cutanées selon l'invention englobent, sans y être limitées, la rosacée, le psoriasis, le prurit, l'eczéma tel que l'eczéma de contact et la dermatite atopique. Au sens de l'invention, l'eczéma comprend l'eczéma atopique (également appelé dermatite ou dermite atopique), l'eczéma de contact et l'eczéma nummulaire. Une forme préférée d'eczéma est la dermatite atopique.

Une forme préférée de prurit est un prurit associé à une dermatose inflammatoire et/ou à une lésion cutanée.

La rosacée comprend, sans y être limitée, la rosacée érythématotélangiectasique (sous-type 1), la rosacée papulopustulaire (sous-type 2), la rosacée phymateuse (sous-type 3) et la rosacée oculaire (sous-type 4) et les variantes granulomateuses de la rosacée.

Dans un mode de réalisation préféré, la pathologie inflammatoire cutanée est la rosacée érythématotélangiectasique (sous-type 1) ou la rosacée papulopustulaire (sous-type 2).

Au sens de l'invention, on entend par « diagnostic » une méthode permettant de déterminer si un individu est effectivement atteint par une pathologie cutanée inflammatoire. On entend par « pronostic » une méthode permettant de prédire l'évolution d'une maladie inflammatoire cutanée chez un patient qui en est atteint, éventuellement en présence d'un traitement thérapeutique. On entend par « prédiction d'une maladie cutanée inflammatoire » une méthode permettant d'évaluer si un individu est susceptible de développer ladite maladie cutanée inflammatoire.

Typiquement, les utilisations selon l'invention sont des utilisations *ex vivo,* plus exactement *in vitro.* En particulier, ces utilisations ne couvrent pas des utilisations dans lesquelles l'anticorps serait en interaction directe avec le corps humain ou un animal. Les utilisations selon l'invention comprennent généralement la détection ou la quantification de Nav 1.9 dans un échantillon biologique du patient à l'aide d'un anticorps selon l'invention. En d'autres termes, les utilisations selon l'invention comprennent généralement la mise en contact d'un échantillon biologique du patient dans des conditions permettant la formation d'un complexe immun entre Nav 1.9, potentiellement présent dans l'échantillon, et l'anticorps dirigé contre Nav 1.9, ce par quoi l'expression de Nav 1.9 dans l'échantillon biologique peut être détectée et/ou quantifiée. Comme cela sera expliqué ci-après, l'échantillon biologique peut subir un certain nombre de traitements avant sa mise en contact avec l'anticorps dirigé contre Nav 1.9.

La présente invention concerne également une méthode *in vitro* de diagnostic d'une maladie cutanée inflammatoire, chez un individu comprenant la détection de Nav 1.9, à l'aide d'un anticorps selon l'invention, dans un échantillon biologique de l'individu. Plus précisément, cette méthode peut comprendre les étapes consistant à:
(a) détecter ou quantifier l'expression de Nav1.9 dans un échantillon biologique du patient avec un anticorps selon l'invention, et
(b) comparer l'expression de Nav1.9 obtenue à l'étape (a) avec l'expression de Nav1.9 détectée ou quantifiée dans un ou plusieurs échantillons biologiques provenant d'un ou plusieurs sujets contrôles.

Dans certains modes de réalisations, le ou les sujets contrôles sont des individus souffrant de la maladie cutanée inflammatoire que l'on souhaite diagnostiquer chez le patient. Dans ce mode de réalisation, on peut considérer que le patient souffre de la maladie cutanée inflammatoire si l'expression de Nav 1.9 déterminée à l'étape (a) est au moins égale à celle des sujets contrôles. Si l'expression de Nav 1.9 déterminée à l'étape (a) est significativement inférieure à celle détectée pour les sujets contrôles, le diagnostic est négatif. Enfin, si l'expression de Nav 1.9 déterminée à l'étape (a) n'est que très légèrement inférieure à celle déterminée pour les sujets contrôles, le patient peut être diagnostiqué comme un patient à risque, c'est-à-dire un patient susceptible de développer la maladie cutanée inflammatoire.

Dans d'autres modes de réalisation, le ou les sujets contrôles correspondent à des patients sains, c'est-à-dire à des individus qui ne souffrent pas de la maladie cutanée inflammatoire. Dans ces modes de réalisations, le patient est diagnostiqué comme souffrant de la maladie cutanée inflammatoire, ou susceptible d'en souffrir, si son échantillon biologique présente une expression de Nav 1.9 supérieure à celle du ou des échantillons provenant des patients sains. En revanche, si l'expression de Nav 1.9 déterminée à l'étape (a) est inférieure ou égale à celle des échantillons provenant des patients contrôles (sains), alors le diagnostic peut être considéré comme négatif. On peut considérer que l'échantillon du patient est positif si l'échantillon présente une expression pour Nav 1.9 au moins 10% supérieure, de préférence au moins 20% supérieure à celui du ou des échantillons des patients sains.

La détection ou la quantification de Nav 1.9 dans les échantillons biologiques du ou des sujets contrôles peuvent être concomitantes à celles effectuées pour l'échantillon du patient ou provenir de données collectées antérieurement et disponibles, par exemple, sur une base de données.

L'invention a également pour objet l'utilisation d'un anticorps selon l'invention pour évaluer l'efficacité d'un médicament dans le traitement d'une maladie cutanée inflammatoire. Afin de suivre l'efficacité d'un traitement thérapeutique d'une maladie cutanée inflammatoire chez un patient, on peut mettre en oeuvre les étapes suivantes:
(a) détecter ou quantifier l'expression de Navl.9, à l'aide de l'anticorps selon l'invention, dans un échantillon biologique du patient avant traitement,
(b) détecter ou quantifier l'expression de Navl.9, à l'aide de l'anticorps selon l'invention, dans un échantillon biologique du patient après traitement, et
(c) comparer l'expression de Nav1.9 de l'étape (a) avec l'expression de Nav1.9 de l'étape (b).

L'efficacité du traitement est déterminée en comparant les expressions de Nav 1.9 dans les échantillons (a) et (b). Si l'expression de Nav 1.9 est plus faible dans l'échantillon de l'étape (b) que dans l'échantillon de l'étape (a), ceci signifie que le traitement est efficace. S'il y a une augmentation de l'expression de Nav 1.9, on peut considérer que le traitement est peu actif ou inactif selon l'importance de la variation observée. Dans certains modes réalisations, on considère que le traitement a un effet thérapeutique sur la maladie cutanée inflammatoire s'il implique une diminution d'expression de Nav 1.9 d'au moins 10%, de préférence d'au moins 20%, de manière encore plus préférée d'au moins 50%.

De préférence, dans les deux étapes (a) et (b), on utilise le même anticorps selon l'invention. Un autre objet selon l'invention est l'utilisation d'un anticorps selon l'invention pour le suivi *in vitro* de l'évolution d'une maladie cutanée inflammatoire. Pour ce faire, les étapes suivantes peuvent être réalisées :
(a) détecter ou quantifier l'expression de Navl.9, à l'aide d'un anticorps selon l'invention, dans un premier échantillon biologique du patient à un temps t1,
(b) détecter ou quantifier l'expression de Navl.9, à l'aide d'un anticorps selon l'invention, dans un second échantillon biologique du patient, à un temps t2 postérieur au temps t1, et
(c) comparer l'expression de Navl.9 de l'étape (b) avec l'expression de Navl.9 de l'étape (a).

De préférence, dans les deux étapes (a) et (b), on utilise le même anticorps selon l'invention.

La comparaison des taux d'expressions de Nav 1.9 dans l'étape (c) est un critère permettant de déterminer la progression ou le stade de la maladie cutanée inflammatoire. Il est ainsi possible de déterminer si la maladie est en régression, stationnaire ou s'aggrave.

Si l'échantillon de l'étape (b) présente un taux d'expression de Nav 1.9 inférieur au taux d'expression mesuré à l'étape (a), il peut être conclu que la rosacée est en régression. A l'inverse, si l'échantillon de l'étape (b) présente un taux d'expression de Nav 1.9 supérieur à celui mesuré à la première étape, la rosacée est en voie de progression. Le temps t2 peut être postérieur d'au moins 1 mois, de préférence d'au moins 6 mois, voire d'au moins 1 an au temps t1. L'invention a également pour objet l'utilisation d'un anticorps selon l'invention pour générer des données et/ou des informations utiles pour diagnostiquer, et/ou pour prédire et/ou pour suivre l'évolution d'une maladie cutanée inflammatoire, ces méthodes pouvant contenir l'une quelconques des combinaisons d'étapes ci-dessus décrites.

Dans l'ensemble des méthodes et utilisations ci-dessus décrites, la pathologie cutanée inflammatoire peut être choisie parmi la rosacée, le psoriasis, le prurit, l'eczéma de contact et la dermatite atopique. De préférence, il s'agit de la rosacée, par exemple la rosacée de sous-type 1 (rosacée érythématotélangiectasique), la rosacée de sous-type 2 (rosacée papulopustulaire), la rosacée ou une variante granulomateuse de rosacée.

Dans l'ensemble des utilisations et méthodes ci-dessus décrites, le ou les échantillons biologiques sont de préférence des échantillons de peau. Il peut s'agir d'échantillon de derme ou de derme-épiderme ou encore d'épiderme. De préférence, il s'agit d'un échantillon de peau comprenant des fibres sensitives nerveuses. A titre d'exemple, il peut s'agir d'une biopsie de peau de taille variable typiquement de 0,1 à 1 mm de diamètre. Comme cela est détaillé ci-après, l'échantillon biologique peut subir un ou plusieurs traitements afin de permettre la détection ou la quantification de Nav 1.9 par un anticorps selon l'invention.

Les méthodes et utilisations selon l'invention sont basées sur une étape de mise en contact de l'échantillon biologique avec l'anticorps selon l'invention dans des conditions permettant la formation d'un complexe immun entre Nav 1.9, potentiellement présent dans l'échantillon, et ledit anticorps. La détection et/ou la quantification de Nav 1.9 dans l'échantillon est en général réalisée via la formation et éventuellement la détection du complexe immun.

La détection et/ou la quantification de Nav 1.9 à l'aide d'un anticorps selon l'invention peut être réalisée par toute méthode connue de l'homme du métier, par exemple par immunodosage, par western blot ou par immunomarquage, en particulier par immunohistochimie ou par immunocytochimie. A titre d'exemple de techniques d'immuno-dosages, on peut citer les dosages radio-immunologiques (RIA), les dosages immunologiques « magnétiques » (magnetic immunoassay MIA), les tests ELISA en sandwich ou par compétition. La quantification et/ou la détection de Nav 1.9 dans un échantillon biologique peut être basée sur une étape séparative par immuno-chromatographie dans laquelle l'anticorps selon l'invention est utilisé en tant que ligand d'affinité. Il peut être envisagé de détecter ou de quantifier Nav 1.9 dans un échantillon biologique par immunoprécipitation ou encore par résonance plasmonique de surface dans laquelle l'anticorps selon l'invention est immobilisé à la surface du senseur.

Les techniques préférées sont l'immunohistochimie et le western blot. Il s'agit là de techniques bien connues de l'homme du métier. Des exemples de mises en oeuvre sont présentés dans la partie expérimentale de la présente demande.

Brièvement, la technique par western blot comprend la préparation d'un extrait protéique à partir de l'échantillon biologique, la mise en oeuvre d'une étape d'électrophorèse généralement en conditions dénaturantes (gel SDS-PAGE), le transfert des protéines ayant migré sur une membrane puis une étape d'immunodétection à l'aide d'un anticorps primaire (anticorps anti-Nav 1.9 selon l'invention) puis une étape de révélation du complexe anticorps-Nav 1.9 éventuellement formé.

Pour la détection ou la quantification de Nav 1.9 dans un échantillon biologique par western blot, on préfère utiliser un anticorps selon l'invention dirigé contre un épitope inclus dans un peptide de séquence SEQ ID N°1 ou SEQ ID N°3.

La technique d'immunohistochimie comprend la préparation d'une coupe de tissu, de préférence de derme, qui peut être soit cryofixée, soit fixée chimiquement. La coupe de tissu peut être ensuite incluse dans une résine et/ou réhydratée puis incubée avec l'anticorps selon l'invention dans des conditions favorables à la formation du complexe anticorps-Nav 1.9. La détection du complexe peut être effectuée directement si l'anticorps est couplé à un moyen de détection ou, le cas échéant à l'aide d'un anticorps secondaire. En général, le moyen de détection couplé à l'anticorps selon l'invention, ou le cas échéant, à l'anticorps secondaire est une molécule fluorescente. La détection des complexes Nav 1.9- anticorps selon l'invention peut alors être effectuée par microscopie à fluorescence. Dans certains modes de réalisation, afin de visualiser les structures cellulaires au niveau desquelles une expression de Nav 1.9 est attendue, un co-marquage avec un second anticorps peut être effectué. A titre d'exemple, afin de visualiser les fibres nerveuses sensitives du derme, un co-marquage avec un anticorps anti-périphérine peut être effectué. Dans un mode de réalisation préféré, pour détecter ou quantifier Nav 1.9 dans un échantillon de peau par immunomarquage, on utilise un anticorps dirigé contre un épitope inclus dans un peptide de séquence SEQ ID N°2. L'échantillon de peau est de préférence une coupe de derme cryofixée.

Dans certains modes de réalisations, les méthodes utilisations selon l'invention peuvent comprendre la détection d'un marqueur supplémentaire à Nav 1.9 ou l'évaluation d'un symptôme particulier de la maladie inflammatoire cutanée. A titre d'exemple, lorsque la maladie inflammatoire cutanée est la rosacée, les méthodes selon l'invention peuvent comprendre la détection d'un marqueur supplémentaire ou d'un symptôme de la rosacée tel que les papules, l'hyperréactivité vasculaire, l'érythème, ou les sensations de brûlures, picotements ou cisaillements.

Enfin, l'invention concerne également un kit, ou l'utilisation dudit kit, pour le diagnostic d'une maladie cutanée inflammatoire; Le kit comprend un anticorps selon l'invention et en option un moyen de détection dudit anticorps. Le kit diagnostic peut également comprendre un ou plusieurs objets supplémentaires, par exemple, une notice explicative pour la mise en oeuvre du test diagnostic, un ou plusieurs réactifs pour la préparation de l'échantillon biologique, ou encore un ou plusieurs moyens utiles pour le prélèvement de l'échantillon biologique.

La pathologie cutanée inflammatoire peut être choisie parmi la rosacée, le psoriasis, le prurit, l'eczéma de contact et la dermatite atopique. De préférence, il s'agit de la rosacée, par exemple la rosacée de sous-type 1 (rosacée érythématotélangiectasique), la rosacée de sous-type 2 (rosacée papulopustulaire), ou une variante granulomateuse de rosacée.

D'autres aspects et avantages de la présente invention apparaîtront à la lecture des exemples qui suivent. Ces exemples doivent être considérés comme illustratifs et en aucun cas comme limitant la portée de l'invention.

### Exemples

### Exemple 1 : préparation d'anticorps polyclonaux

### Anticorps Rbaa4 et Rbaa6 :

Deux lapins ont été, chacun, immunisés avec un mélange peptidique contenant les 2 peptides (SEQ ID N°1 et SEQ ID N°3) couplés à KLH (calendrier ci-après).

Contrôle de la réponse immunitaire par ELISA. Purification sur colonne d'affinité (couplage Hi-trap NHS) des prélèvements J63 et J77.

L'anticorps Rbαa4 a été isolé par un procédé de purification comprenant une étape de chromatographie d'affinité dans laquelle le ligand immobilisé était le peptide de SEQ ID N°1. L'anticorps Rbαa6 a été isolé par un procédé de purification comprenant une étape de chromatographie d'affinité dans laquelle le ligand immobilisé était le peptide de SEQ ID N°3.

### Anticorps 3881.2 et 3881.1 :

Deux lapins ont été, chacun, immunisés avec un mélange peptidique contenant les 2 peptides (SEQ ID N°2 et SEQ ID N°4) couplés à KLH (calendrier ci-après). Purification sur colonne d'affinité (AF-Amino TOYOPEARL/MBS) des prélèvements J28. L'anticorps 3881.1 a été isolé par un procédé de purification comprenant une étape de chromatographie d'affinité dans laquelle le ligand immobilisé était le peptide de SEQ ID N°2. L'anticorps 3881.2 a été isolé par un procédé de purification comprenant une étape de chromatographie d'affinité dans laquelle le ligand immobilisé était le peptide de SEQ ID N°4.

| **Référence anticorps** | **Peptide antigénique** | **Nbre d'immunisations** | **Jour de prélèvement/purification** |
|---|---|---|---|
| Rbaa4 | N-terminale de Nav1.9 (SEQ ID N°1) | 5 (J0, J14, J28, J42, J56) | J63-J77 |
| 3881.1 | Peptide de la boucle intracellulaire I-II (SEQ ID N°2) | 4 (J0, J7, J10, J18) | J28 |
| 3881.2 | Peptide de la boucle intracellulaire II-III (SEQ ID N°4) | 4 (J0, J7, J10, J18) | J28 |
| Rbaa6 | Peptide de la boucle intracellulaire II-III (SEQ ID N°3) | 5 (J0, J14, J28, J42, J56) | J63-J77 |

### Exemple 2 : Caractérisations des anticorps

### 1. Immunofluorescence sur systèmes hétérologues

Un système d'expression hétérologue a été utilisé pour sélectionner les anticorps et tester leur spécificité. Des cellules HEK-293 ont été transfectées : 1) avec un plasmide contenant la séquence nucléique codant pour la sous-unité alpha Nav1.9 humaine étiquetée *myc* à son extrémité C-terminale ou 2) avec les séquences codant pour Nav 1.5 ou Nav 1.7 humains (hNav1.5 et hNav1.7). Ces derniers n'étant pas étiquetés, ils ont été co-transfectés avec un plasmide codant pour l'expression de la GFP..

24 h après transfection, les cellules ont été fixées au paraformaldéhyde 2% (PAF), perméabilisées au triton 0,1% puis incubées avec l'anticorps anti-hNav 1.9 à tester. L'expression de hNav1.9 est testée par un co-marquage avec un anticorps monoclonal anti-myc alors que l'expression de hNav1.5 et hNav1.7 est testée avec un anticorps PanNav, reconnaissant tous les canaux sodiques.

### - Résultats

Le marquage du système hétérologue exprimant hNav1.9-myc avec l'anticorps Rbaa4 ou l'anticorps Rbαa6 est co-localisé avec le marquage anti-Myc. On ne distingue aucun marquage par Rbαa4 et Rbaa6 des cellules non-transfectées, ce qui montre que ces anticorps ne se lient pas à une protéine endogène. A titre d'illustration, la Figure 1 montre les résultats de comarquage avec l'anticorps monoclonal anti-Myc et l'anticorps polyclonal Rbαa4 pour des cellules recombinantes exprimant hNav1.9-Myc (Figure 1A) et des cellules non-recombinantes (Figure 1B). Des marquages identiques ont été obtenus pour l'anticorps Rbαa6 (donnée non montrée).

Il apparait également que les anticorps Rbαa4 et Rbαa6 ne marquent pas les systèmes hétérologues exprimant hNav 1.5 et hNav 1.7, dont le pourcentage d'identité est de 56 % et 55 % avec hNav1.9. Ceci montre que Rbαa4 et Rbαa6 n'ont pas de réactivité croisée vis-à-vis de hNav 1.5 et hNαv1.7. A cet égard, la Figure 2 montre les résultats d'immunomarquage avec l'anticorps Rbaa4 des cellules HEK exprimant hNav1.5, identifiées par la GFP (Figure 2A) et des cellules HEK exprimant hNav1.7, identifiées par la GFP (Figure 2B). Alors que le signal émis par la GFP est clairement visible, le marquage avec Rbαa4 des cellules est très faible voire inexistant. Le marquage avec un anticorps monoclonal anti-PanNav a confirmé une expression correcte de hNav 1.5 ou hNav 1.7 par les cellules HEK. Des marquages identiques ont été obtenus pour l'anticorps Rbaa6 (données non montrées). Les anticorps Rbαa4 et Rbαa6 reconnaissent donc de manière spécifique hNav 1.9 et ne présentent pas de réactivité croisée avec hNav 1.5 et hNav 1.7.

Aucun marquage n'est détecté avec l'anticorps 3881.2 dirigé contre le peptide de SEQ ID N°4 que ce soit avec les cellules transfectées ou non-transfectées. Cette absence de détection résulte du procédé de fixation chimique qui doit induire une altération de la conformation de la région de hNav 1.9 reconnue par cet anticorps. En revanche, cet anticorps marque de manière très efficace et sensible des tissus non-fixés chimiquement exprimant de manière endogène Nav 1.9 (voir ci-après).

L'anticorps dirigé contre le peptide de SEQ ID N°4 est incapable de détecter hNav.1.9, quel que soit le système cellulaire et le tissu analysé.

### 2. Western Blot

Des analyses par western blot d'extraits protéiques issus de la lyse de cellules HEK transfectées avec hNav1.9-Myc ont été réalisés afin de conforter la spécificité de détection des anticorps Rbαa4 et Rbαa6.

### - Résultats

Les anticorps Rbαa4 et Rbαa6 reconnaissent une bande ayant un poids moléculaire aux alentours de 250 kDa (**Figure 2**). Cette bande est aussi présente lors de la détection avec l'anticorps anti-Myc. De plus, cette bande n'est pas détectée pour l'extrait provenant de cellules non transfectées (contrôle négatif). Le poids moléculaire de la bande spécifique est un peu plus haut que le poids moléculaire attendu pour hNav 1.9-Myc (210 kDa environ). Cette différence de poids moléculaire est certainement attribuable à des modifications post-traductionnelles, en particulier à des glycosylations. Une expérience de déglycosylation sur le lysat protéique a été réalisée et a mis en évidence une diminution de l'intensité de la bande à 250 kDa, ce qui conforte cette hypothèse.

### 3. Immunohistochimie sur tissus humains

Des essais d'immunomarquage de ganglions myentériques, de pulpe dentaire humaine et de ganglions trijumeaux ont été réalisés à l'aide des anticorps Rbαa4, Rbαa6 et 3881.1. Les échantillons ont été préparés de la manière suivante :
Ganglions myentériques : Les résidus opératoires de côlon sont récupérés rapidement après prélèvement, placés sur glace dans du Krebs carbogéné, additionné de bloqueurs de la contraction musculaire (atropine, 2 µM et nicardipine, 6 µM), avant dissection.

Composition du Krebs carbogéné (en mM) : 118 NaCl, 4.8 KCl, 1 NaH2PO4, 25 NaHCO3, 1.2 MgCl2, 2.5 CaCl2 et 11 Glucose, équilibré à pH 7.4 avec 95% O2-5% CO2.

La dissection consiste à enlever la muqueuse, la sous-muqueuse et à réduire l'épaisseur du tissu en enlevant un peu de muscle (longitudinal et circulaire) (Osorio et Delmas, 2010, Nature Protocols, 6(1) :15-27).

Le tissu est ensuite incubé 1h dans du PBS 4% sucrose (masse/volume) à 4°C, puis 12h dans du PBS 20% sucrose, toujours à 4°C.

Composition du PBS (en mM) : 140 NaCl, 10 mM PO4 Buffer (NaHPO4 et NaH2PO4), 3 mM KCI, pH 7.2.

Pulpe dentaire : Après prélèvement, la dent de sagesse est conservée dans du PBS à 4C. La pulpe dentaire est extraite de l'émail, puis incubé 1h dans du PBS 4% sucrose (masse/volume) à 4°C, puis 12h dans du PBS 20% sucrose, toujours à 4°C.

Ganglion trijumeau : Après prélèvement (12-24h post-mortem), les ganglions sont incubés 2h dans du PBS 4% sucrose (masse/volume) à 4°C, puis 12h dans du PBS 20% sucrose, toujours à 4°C.

Enfin, les tissus sont inclus dans de l'OCT et congelés dans de l'isopentane rafraîchi avec de la carboglace. Les blocs sont stockés au -80°C.

Les coupes transverses de 15-30 µm sont faites au cryostat, montées sur des lames Superfrost, séchées (1h sous Sorbonne) puis stockées au -80°C jusqu'à utilisation.

A la sortie du congélateur, les lames sont séchées 30 min sous une Sorbonne.

Les tissus peuvent ensuite être traités de deux manières différentes selon que l'anticorps primaire nécessite un tissu « frais » ou « fixé ». Ils sont donc :
- Réhydratées avec du PBS pendant 1h : « tissu cryofixé »
- ou fixés avec du paraformaldéhyde (PAF) 4% (volume/volume) pendant 10 min, puis rincées 4 x 10 min dans du PBS : « tissu chimiquement fixé »

### ▪ Protocole d'immunomarquage

Les coupes sont incubées 1h30 à température ambiante dans un milieu de saturation : PBS + 3% (masse/volume) BSA + 0,3% (volume/volume) Triton X-100. Les anticorps primaires sont ajoutés au milieu de saturation pour une incubation de 12 h à 4°C en chambre humide. Les coupes sont ensuite rincées 4 fois 15 min dans du PBS avant 45 min d'incubation à à température ambiante avec les anticorps secondaires dans du PBS + 3% (masse/volume) BSA en chambre humide. Enfin, les coupes sont rincées 4 fois 15 min dans du PBS avant d'être montées dans un milieu de montage (Mowiol ou Prolong Gold)

Un co-marquage avec un anticorps anti-HuC/D ou anti-NF200 pour les ganglions myéntériques, avec un anticorps anti-périphérine pour la pulpe dentaire et avec un anticorps anti-NF200 ont été réalisés afin de visualiser les neurones et les fibres nerveuses.

### ▪ Résultats

Les anticorps Rbαa4 et Rbαa6 donnent un marquage intéressant pour les différents tissus humains, qu'ils soient chimiquement ou cryo-fixés (données non montrées). L'anticorps 3881.1 marque de manière très efficace et sensible les tissus cryo-fixés, en particulier de pulpe dentaire et du plexus myentérique, connus pour exprimer Nav 1.9. En revanche, il est inefficace sur tissu fixé chimiquement (données non montrées).

Ces expériences d'immunomarquage ont donc confirmé la possibilité d'utiliser les anticorps Rbαa4, Rbαa6 et 3881.1 comme outil pour explorer l'expression tissulaire de hNav 1.9.

### Exemple 3 : Expression de hNavl.9 par les fibres nerveuses sensitives cutanées

La détection de l'expression de Nav1.9 au niveau des fibres nerveuses cutanées a été explorée par marquage par immunofluorescence à l'aide d'anticorps polyclonaux dirigés contre la sous-unité alpha du canal sodium voltage-dépendant Nav1.9 humain, à savoir les anticorps Rbαa6, Rbαa4 et l'anticorps 3881.1.

Un co-marquage à l'aide d'un anticorps dirigé contre la périphérine, une protéine spécifique du tissu nerveux périphérique, a été réalisé afin de localiser les fibres nerveuses.

Différents types d'échantillons de peau ont été utilisés : derme profond, derme, interface derme-épiderme et follicule pileux.

### - Préparation des échantillons

Les échantillons de peau ont été préparés de la manière suivante :
Après désinfection de la peau, des biopsies de peau de 3 mm ont été réalisées chez un patient soumis à une anesthésie locale au site de la biopsie. La biopsie est lavée dans du PBS pH7.2 et placée dans un tube épendorf dans de l'azote liquide. L'épendorf est ensuite stocké à -80°C jusqu'à inclusion en matrice OCT. Des coupes de congélation de 10 µm d'épaisseur sont ensuite réalisées.

### - Protocole de marquage par immunofluorescence

Les coupes sont incubées 1h30 à température ambiante dans un milieu de saturation : PBS + 3% (masse/volume) BSA + 0,3% (volume/volume) Triton X-100. Les anticorps primaires sont ajoutés au milieu de saturation pour une incubation de 12 h à 4°C en chambre humide. Les coupes sont ensuite rincées 4 fois 15 min dans du PBS avant 45 min d'incubation à à température ambiante avec les anticorps secondaires dans du PBS + 3% (masse/volume) BSA en chambre humide. Enfin, les coupes sont rincées 4 fois 15 min dans du PBS avant d'être montées dans un milieu de montage (Mowiol ou Prolong Gold).

### - Résultats

Les Figures 4 et 5 montrent le marquage par immuno fluorescence de coupe de dermes profond par l'anticorps anti-périphérine (Figures 4A et 5A), l'anticorps Rbαa6 (Figure 4B), et l'anticorps 3881.1 (Figure 5B). Les résultats d'immunomarquage obtenus par les anticorps Rbaa6 et 3881.1 sont similaires. Des résultats d'immunomarquage analogues du derme ont été obtenus avec l'anticorps Rbαa4 (donnée non montrée). On observe une expression du canal sodique Nav1.9 localisée au niveau des fibres sensitives du derme profond. Des zones de co-localisation nette de la périphérine et de Nav1.9 sont mises en évidence dans les Figures 4C et 5C.

Les Figures 6 et 7 montrent les résultats d'immuno-marquage de la périphérine et de Nav1.9 au niveau de l'interface derme-épiderme et d'un follicule pileux respectivement. Ces clichés d'immuno-marquage ont été obtenus avec l'anticorps 3881.1.

La Figure 6 montre que l'expression localisée de Nav1.9 au niveau d'une fibre nerveuse isolée du derme. Un résultat analogue est observé au niveau du follicule pileux où Nav1.9 est exprimée, de manière localisée, par les fibres sensitives innervant le follicule (Figure 7). Aucun marquage n'a été détecté dans le derme.

### - Conclusions

Ces expériences d'immuno-marquage montrent que le canal sodique Nav1.9 est exprimé au niveau des fibres sensitives cutanées, aussi bien dans l'épiderme et que dans le derme profond. Les anticorps selon l'invention se révèlent êtres des outils de choix pour la détection et la quantification de Nav1.9 dans les échantillons de peau.

### Exemple 4 : Surexpression de Navl.9 par les fibres sensitives cutanées chez des patients atteints de rosacée.

### Préparation des échantillons

Les échantillons de peau (derme et follicule pileux) ont été préparés comme décrit dans l'exemple 1: Après désinfection de la peau, des biopsies de peau de 3 mm ont été réalisées chez un patient atteint de Rosacée et soumis à une anesthésie locale au site de la biopsie. La biopsie est lavée dans du PBS pH7.2 et placée dans un tube épendorf dans de l'azote liquide. L'épendorf est ensuite stocké à -80°C jusqu'à inclusion en matrice OCT. Des coupes de congélation de 10 µm d'épaisseur sont ensuite réalisées.

### Protocole de marquage par immunofluorescence

Le protocole d'immunomarquage est similaire à celui décrit pour l'Exemple 1

Les anticorps suivants ont été utilisés :
▪ Pour la périphérine, l'anticorps primaire est un anticorps monoclonal (Millipore, MAB1527) de souris et l'anticorps secondaire est un anticorps anti-souris d'âne couplé à l'Alexa488 (Invitrogen, A-21202).
▪ Pour Nav1.9, l'anticorps primaire est l'anticorps polyclonal 3881.1 de lapin et l'anticorps secondaire est un anticorps anti-lapin d'âne couplé à TRITC (Jackson Immunoresearch).

### Résultats

Les Figures 8, 9 et 10 montrent les résultats d'immuno-marquage de la périphérine et de Nav1.9 au niveau du derme, d'un follicule pileux et de l'interface derme/épiderme provenant d'échantillons de peau atteints par la rosacée. Nav1.9 est localisé au niveau des fibres sensitives du derme et du follicule pileux. Alors qu'un marquage relativement homogène a été observé pour les échantillons de peau saine, on observe la présence de clusters d'expression c'est-à-dire de zones localisées (spots) présentant une expression très élevée de Nav 1.9. La Figure 10 montre par ailleurs une expression de Nav 1.9 dans l'épiderme, ce qui n'avait pas été observé pour les échantillons sains.

Ces résultats préliminaires montrent donc une surexpression de Nav 1.9 au niveau d'échantillons de peau atteints par la rosacée.

### Exemple 5 : Mise en évidence de l'implication du canal sodique Navl.9 dans la réponse neuro-inflammatoire.

L'objectif de cette étude était de mettre en évidence l'impact de Nav1.9 sur la libération du peptide relié au gène calcitonine (ou calcitonin gene-related peptide (CGRP)). Le peptide CGRP est un puissant vasodilatateur et un médiateur de la douleur au niveau système nerveux périphérique et central. CGRP est également impliqué dans l'inflammation neurogénique périphérique, en particulier de la peau. A cette fin, les Demandeurs a comparé la libération de CGRP par des cultures primaires de neurones, après ou sans traitement à la capsaïcine. Les cultures primaires de neurones ont été obtenues à partir de ganglions spinaux (Dorsal Root Ganglion-DRG) prélevés chez des souris sauvages (WT) et des souris chez lesquelles le gène du canal sodique Nav1.9 a été invalidé (souris KO pour Nav1.9).

### - Cultures primaires de neurones provenant de ganglions spinaux de souris adultes.

Les prélèvements de ganglions spinaux ont été réalisés chez des souris C57BL6 mâles âgées de 2 à 3 mois. En moyenne, 20 ganglions spinaux ont été prélevés par souris, ce qui correspond à un total de 50 000 et 80 000 neurones/souris.

Les neurones collectés ont été ensemencés dans des plaques 96 puits à raison de 10 000 cellules par puits. Les puits ont été préalablement recouverts de poly-L-Lysine (12µg/cm²) et laminine (6µg/cm²) pour favoriser l'adhésion des neurones. Les cellules ont été incubées 24h à 37°C avant le début du test. dans un milieu de culture adapté (D-MEM supplémenté en glucose, NEAA,L-glutamine, pyruvate, NGF (Nerve Growth Factor 2.5s mouse submaxillary) GDNF (Glial cell-line Derived Neurotrophic Factor) et sérum de veau foetal).

Au total, les cultures cellulaires ont été obtenues à partir de 12 souris (6 WT et 6 KO). 5 à 8 puits de culture primaire de neurones ont été préparés par souris.

### - Quantification de la libération de CRGP dans le milieu après traitement à la capsaïcine

Une partie des cultures primaires (3 à 5 puits de culture par souris) ont été incubées 15 min en présence de capsaïcine à 300 nM. La seconde partie des cultures primaires (2 à 3 puits de culture par souris) n'ont subi aucun traitement.

Le dosage de CGRP libéré dans le surnageant a été réalisé par dosage ELISA en utilisant le Kit SPIbio ref A05482 destiné au dosage du CGRP de rat, mais qui est utilisable également pour quantifier le CRGP de souris.

Brièvement, les surnageants des cultures primaires ont été prélevés et dilués au 1/5 avant d'être rajoutés dans les puits d'une microplaque recouverts par un anticorps monoclonal anti-CGRP. Un second anticorps anti-CGRP (polyclonal), couplé à l'acétylcholine estérase, a été ensuite rajouté. Après incubation et rinçage, la détection a été réalisée à l'aide du réactif d'Ellman (DTNB) par mesure de l'absorbance à 405 ou 414 nm.

### - Résultats

Les résultats sont illustrés par les Figure 11A et 11B.

Chez les cultures primaires obtenues à partir de souris sauvages (WT), on observe que le traitement à la capsaïcine a entraîné une augmentation significative de la quantité de CGRP (+43,5%) présente dans le surnageant. En revanche, aucune variation de la quantité de CGRP libéré dans le surnageant n'a été observée pour les cultures primaires provenant de souris KO : la quantité de CGRP détecté dans le surnageant est équivalente au niveau basal, avant stimulation capsaïcine, de CGRP du surnageant des cellules WT.

Nav1.9 est donc impliqué dans la régulation de la libération de CGRP consécutivement à une stimulation à la capsaïcine.

### Exemple 6: Quantification du pool intracellulaire de CGRP chez des cultures neuronales obtenues à partir de souris sauvages (WT) et de souris KO pour Navl.9 (KO).

### - Cultures primaires de neurones provenant de ganglions spinaux de souris adultes.

Le protocole utilisé est identique à celui de l'Exemple 3. Les cultures neuronales primaires ont été préparées à partir de 4 souris KO et 4 WT.

### - Dosage du pool intracellulaire de CGRP.

Après incubation 24h à 37°C et rinçage, les cultures primaires ont été lysées au Triton X100 à 1%. Le dosage du peptide CGRP dans les lysats cellulaires a été réalisé par le test Elisa décrit dans l'Exemple 3.

Il s'avère que la concentration moyenne de CRPG pour les cultures primaires provenant de souris KO est légèrement inférieure à celle obtenue pour les cultures primaires provenant des souris sauvage. Néanmoins, la différence observée n'est pas significative.

En conséquence, la différence de relargage de CGRP, sous stimulation capsaïcine, observée pour les cultures neuronales primaires WT et KO, dans l'exemple 3, n'est pas attribuable à un déficit intracellulaire de CGRP chez les cellules neuronales primaires KO.

### Conclusion

Les résultats de libération du peptide CGRP par les cultures neuronales primaires, provenant de souris WT et KO, après ou sans stimulation à la capsaïcine, et les résultats d'immunomarquage de coupes de derme et d'épiderme suggèrent très fortement l'implication de Nav1.9 dans la potentialisation de l'inflammation neurogène observée au niveau de peau dans les pathologies cutanées inflammatoires. Nav1.9 constitue donc un marqueur d'intérêt pour le diagnostic des maladies cutanées inflammatoires, en particulier la rosacée.

**Tableau de séquences**

| **Référence** | **Séquence** |
|---|---|
| SEQ ID N°1 | Peptide inclus dans l'extrémité N-terminale de hNav1.9 : |
| | IAIQKEKKKSKDQTGEV |
| SEQ ID N°2 | Peptide de la boucle intracellulaire I-II |
| | ESGKDQPPGSDSDEDC |
| SEQ ID N°3 | Peptide de la boucle intracellulaire II-III |
| | QAYELHQENKKPTSQRV |
| SEQ ID N°4 | Peptide de la boucle intracellulaire II-III |
| | SNEERNGNLEGEARK |

## Revendications

1. Anticorps dirigé contre Nav 1.9 humain **caractérisé en ce que** ledit anticorps se lie à un épitope inclus dans un peptide de séquence SEQ ID N°1, SEQ ID N°2 ou SEQ ID N°3.

2. Anticorps dirigé contre Nav 1.9 humain selon la revendication 1 en ce qu'il est adapté à une utilisation pour la détection ou la quantification de Nav 1.9 dans un échantillon biologique, de préférence un échantillon de peau.

3. Anticorps dirigé contre Nav 1.9 humain selon la revendication 1 ou 2, ledit anticorps étant un anticorps polyclonal.

4. Anticorps dirigé contre Nav 1.9 humain selon la revendication 1 ou 2, ledit anticorps étant choisi parmi un anticorps monoclonal, de préférence une immunoglobuline (Ig), un anticorps à simple domaine (sdab), ou une IgNar, un polypeptide comprenant un domaine variable d'un anticorps tel qu'un ScFv, un VH, un V_{H}H, un V_{NAR}, un Fab, et les versions chimériques ou humanisés de ceux-ci.

5. Anticorps dirigé contre Nav 1.9 humain selon l'une des revendications 1 à 4 **caractérisé en ce qu'**il ne se lie pas à Nav 1.5 et Nav 1.7.

6. Anticorps dirigé contre Nav 1.9 humain selon l'une des revendications 1 à 5 **caractérisé en ce qu'**il est obtenu à partir d'anticorps ou de cellules exprimant des anticorps provenant d'un animal non-humain immunisé avec un composé antigénique comprenant au moins un peptide de SEQ ID N°1, SEQ ID N°2 ou SEQ ID N°3.

7. Anticorps dirigé contre Nav 1.9 humain selon l'une des revendications 1 à 6 **caractérisé en ce qu'**il est couplé à un moyen de détection, de préférence choisi parmi une enzyme produisant un signal détectable telle que la peroxydase de raifort, la phosphatase alcaline, la 3-galactosidase, ou la glucose-6-phosphate déshydrogénase, un chromophore, un composé fluorescent, un composé luminescent, un radionucléide tel que ³²P, ³⁵S ou ¹²⁵I, une particule métallique, par exemple une nanoparticule d'or, une biotine, une streptavidine, une avidine, un sucre ou une lectine.

8. Méthode de préparation d'un anticorps dirigé contre Nav 1.9 humain comprenant une étape d'immunisation d'un animal non-humain avec un composé antigénique comprenant au moins un peptide de SEQ ID N°1, SEQ ID N°2 ou SEQ ID N°3, dans laquelle le composé antigénique comprend de préférence une protéine porteuse couplée à une ou plusieurs copies dudit peptide.

9. Anticorps dirigé contre Nav 1.9 humain susceptible d'être obtenu selon une méthode telle que définie dans la revendication 8.

10. Utilisation d'un anticorps dirigé contre Nav 1.9 humain tel que défini dans l'une quelconque des revendications 1 à 7 ou 9 pour le diagnostic, le pronostic et/ou la prédiction *in vitro* d'une maladie cutanée inflammatoire.

11. Utilisation d'un anticorps dirigé contre Nav 1.9 humain tel que défini dans l'une quelconque des revendications 1 à 7 ou 9 pour le suivi *in vitro* de l'évolution d'une maladie cutanée inflammatoire ou pour évaluer *in vitro* l'efficacité d'un médicament pour le traitement d'une maladie cutanée inflammatoire.

12. Utilisation selon l'une des revendications 10 ou 11 comprenant la détection ou la quantification de Nav 1.9 dans un échantillon biologique d'un patient, à l'aide dudit anticorps dirigé contre Nav 1.9 humain.

13. Utilisation selon la revendication 12, dans laquelle l'échantillon biologique est un échantillon de peau.

14. Utilisation selon l'une des revendications 10 à 13 dans laquelle la maladie cutanée inflammatoire étant choisie parmi le groupe constitué par la rosacée, le psoriasis, le prurit, l'eczema de contact et la dermatite atopique.

15. Utilisation selon la revendication 14, dans laquelle la maladie cutanée inflammatoire est la rosacée, de préférence choisie parmi le groupe constitué par la rosacée érythèmatotélangiectasique (sous-type I), la rosacée papulopustuleuse (sous-type II), la rosacée phymateuse (sous-type III) et les variantes granulomateuses de la rosacée.

16. Kit pour le diagnostic d'une maladie cutanée inflammatoire comprenant au moins un anticorps dirigé contre Nav 1.9 tel que défini dans l'une des revendications 1 à 7 ou 9 et en option un moyen de détection dudit anticorps

## Patentansprüche

1. Antikörper gerichtet gegen menschliches Nav 1.9, **dadurch gekennzeichnet, dass** besagter Antikörper an ein Epitop bindet, das in einem Peptid der Sequenz SEQ ID NR: 1, SEQ ID NR: 2 oder SEQ ID NR: 3 enthalten ist.

2. Antikörper gerichtet gegen menschliches Nav 1.9 gemäß Anspruch 1, **dadurch gekennzeichnet, dass** er für eine Verwendung für den Nachweis oder die quantitative Bestimmung von Nav 1.9 in einer biologischen Probe, vorzugsweise einer Hautprobe, geeignet ist.

3. Antikörper gerichtet gegen menschliches Nav 1.9 gemäß Anspruch 1 oder 2, wobei besagter Antikörper ein polyklonaler Antikörper ist.

4. Antikörper gerichtet gegen menschliches Nav 1.9 gemäß Anspruch 1 oder 2, wobei besagter Antikörper aus einem monoklonalen Antikörper, vorzugsweise einem Immunglobulin (Ig), einem Einzeldomänen-Antikörper (sdab) oder einem IgNar, einem Polypeptid, umfassend eine variable Domäne eines Antikörpers, wie ein ScFv, eine VH, eine V_{H}H, eine V_{NAR}, ein Fab, und chimäre oder humane Variationen davon, ausgewählt ist.

5. Antikörper gerichtet gegen menschliches Nav 1.9 gemäß einem der Ansprüche 1 bis 4, **dadurch gekennzeichnet, dass** er nicht an Nav 1.5 und Nav 1.7 bindet.

6. Antikörper gerichtet gegen menschliches Nav 1.9 gemäß einem der Ansprüche 1 bis 5, **dadurch gekennzeichnet, dass** er von einem Antikörper oder Antikörper exprimierenden Zellen erhalten wird, die von einem nicht-humanen Tier stammen, das mit einer antigenen Verbindung, umfassend wenigstens ein Peptid der SEQ ID NR: 1, SEQ ID NR: 2 oder SEQ ID NR: 3, immunisiert ist.

7. Antikörper gerichtet gegen menschliches Nav 1.9 gemäß einem der Ansprüche 1 bis 6, **dadurch gekennzeichnet, dass** er an ein Nachweismittel gekoppelt ist, vorzugsweise ausgewählt aus einem Enzym, das ein nachweisbares Signal liefert, wie Meerrettich-Peroxidase, alkalischer Phosphatase, 3-Galactosidase oder Glucose-6-Phosphat-Dehydrogenase, einem Chromophor, einer fluoreszierenden Verbindung, einer lumineszierenden Verbindung, einem Radionuklid wie ³²P, ³⁵S oder ¹²⁵I, einem Metallpartikel, zum Beispiel einem Gold-Nanopartikel, einem Biotin, einem Streptavidin, einem Avidin, einem Zucker oder einem Lektin.

8. Verfahren zur Herstellung eines Antikörpers gerichtet gegen menschliches Nav 1.9, umfassend einen Schritt der Immunisierung eines nicht-humanen Tiers mit einer antigenen Verbindung, umfassend wenigstens ein Peptid der SEQ ID NR: 1, SEQ ID NR: 2 oder SEQ ID NR: 3, wobei die antigene Verbindung vorzugsweise ein Trägerprotein umfasst, das an eine oder mehrere Kopien besagten Peptids gekoppelt ist.

9. Antikörper gerichtet gegen menschliches Nav 1.9, der nach einem wie in Anspruch 8 definierten Verfahren erhalten werden kann.

10. Verwendung eines Antikörpers gerichtet gegen menschliches Nav 1.9, wie in einem der Ansprüche 1 bis 7 oder 9 definiert, für die *in vitro* Diagnose, Prognose und/oder Vorhersage einer entzündlichen Hauterkrankung.

11. Verwendung eines Antikörpers gerichtet gegen menschliches Nav 1.9, wie in einem der Ansprüche 1 bis 7 oder 9 definiert, für die *in vitro* Verfolgung der Entwicklung einer entzündlichen Hauterkrankung oder für die *in vitro* Evaluierung der Wirksamkeit eines Medikaments für die Behandlung einer entzündlichen Hauterkrankung.

12. Verwendung gemäß einem der Ansprüche 10 oder 11, umfassend den Nachweis oder die quantitative Bestimmung von Nav 1.9 in einer biologischen Probe eines Patienten mithilfe besagten Antikörpers gerichtet gegen menschliches Nav 1.9.

13. Verwendung gemäß Anspruch 12, wobei die biologische Probe eine Hautprobe ist.

14. Verwendung gemäß einem der Ansprüche 10 bis 13, wobei die entzündliche Hauterkrankung aus der Gruppe ausgewählt ist, bestehend aus Rosacea, Psoriasis, Pruritus, Kontaktekzem und atopischer Dermatitis.

15. Verwendung gemäß Anspruch 14, wobei die entzündliche Hautkrankheit die Rosacea ist, vorzugsweise ausgewählt aus der Gruppe, bestehend aus Rosacea erythematosa-teleangiectatica (Subtyp I), Rosacea papulopustulosa (Subtyp II), Rosacea phymatosa (Subtyp III) und granulomatösen Varianten der Rosacea.

16. Kit für die Diagnose einer entzündlichen Hauterkrankung, umfassend wenigstens einen Antikörper gerichtet gegen menschliches Nav 1.9, wie in einem der Ansprüche 1 bis 7 oder 9 definiert, und gegebenenfalls ein Mittel zum Nachweis besagten Antikörpers.

## Claims

1. An antibody directed against human Nav 1.9 **characterized in that** said antibody binds to an epitope included in a peptide of sequence SEQ ID No. 1, SEQ ID No. 2 or SEQ ID No.3.

2. The antibody directed against human Nav 1.9 according to claim 1, which is suitable for detecting or quantifying Nav 1.9 in a biological sample, preferably a skin sample.

3. The antibody directed against human Nav 1.9 according to claim 1 or 2, said antibody being a polyclonal antibody.

4. The antibody directed against human Nav 1.9 according to claims 1 or 2, said antibody being selected from a monoclonal antibody, preferably an immunoglobulin (Ig), an antibody with a single domain (sdab), or an IgNar, a polypeptide comprising a variable domain of an antibody, such as ScFv, VH, V_{H}H, V_{NAR}, Fab, and the chimeric or humanized versions thereof.

5. The antibody directed against human Nav 1.9 according to one of claims 1 to 4, **characterized in that** it does not bind to Nav 1.5 and Nav 1.7.

6. The antibody directed against human Nav 1.9 according to one of claims 1 to 5, **characterized in that** it is obtained from antibodies or cells expressing antibodies from a non-human animal immunized with an antigenic compound comprising at least one peptide of SEQ ID No. 1, SEQ ID No. 2 or SEQ ID No. 3.

7. The antibody directed against human Nav 1.9 according to one of claims 1 to 6, **characterized in that** it is coupled with a means for detection, preferably selected from an enzyme producing a detectable signal, such as horseradish peroxidase, alkaline phosphatase, 3-galactosidase or glucose-6-phosphate dehydrogenase, a chromophore, a fluorescent compound, a luminescent compound, a radionuclide, such as ³²P, ³⁵S or ¹²⁵I, a metal particle, for example a gold nanoparticle, a biotin, a streptavidin, an avidin, a sugar or a lectin.

8. A method for preparing an antibody directed against human Nav 1.9 comprising immunizing a non-human animal with an antigenic compound comprising at least one peptide of SEQ ID No. 1, SEQ ID No. 2 or SEQ ID No. 3, preferably wherein the antigenic compound comprises a carrier protein coupled with one or several copies of said peptide.

9. The antibody directed against human Nav 1.9 obtainable according to a method as defined in claim 8.

10. The use of an antibody directed against human Nav 1.9 as defined in any one of claims 1 to 7 or 9 for the diagnostic, the prognosis and/or the prediction *in vitro* of an inflammatory skin disease.

11. The use of an antibody directed against human Nav 1.9 as defined in any one of claims 1 to 7 or 9 for tracking *in vitro* the development of an inflammatory skin disease or for evaluating *in vitro* the effectiveness of a drug for treating an inflammatory skin disease.

12. The use according to one of claims 10 or 11, comprising the detection or the quantification of Nav 1.9 in a biological sample from a patient using said antibody directed against human Nav 1.9.

13. The use according to claim 12, wherein the biological sample is a skin sample.

14. The use according to one of claims 10 to 13 wherein the inflammatory skin disease is selected from the group selected from the group consisting of rosacea, psoriasis, pruritus, contact eczema and atopic dermatitis.

15. The use according to claim 14, wherein the inflammatory skin disease is rosacea, preferably selected from the group consisting of erythematotelangiectatic rosacea (sub-type I), papulopustular rosacea (sub-type II), phymatous rosacea (sub-type III) and granulomatous variants of rosacea.

16. A kit for the diagnostic of an inflammatory skin disease comprising at least one antibody directed against Nav 1.9 as defined in one of claims 1 to 7 or 9 and optionally a means for detecting said antibody.
